# EUROPÄISCHE PATENTANMELDUNG

(11) **EP 3 909 570 A1**
(43) Veröffentlichungstag der Anmeldung: **17.11.2021**
(21) Anmeldenummer: 20213647.9
(22) Anmeldetag: 14.12.2020
(51) Int. Cl.: A61K 31/137, A61K 31/216, A61K 31/222, A61K 31/4025, A61K 31/445, A61K 31/46, A61K 31/4725, A61M 5/178, A61P 13/10

(54) **OXYBUTYNIN FÜR DIE INTRAVESIKALE ANWENDUNG**

(30) Priorität: 15.05.2020 DE 102020002936; 06.07.2020 DE 102020117734
(71) Anmelder: Farco-Pharma GmbH, 50670 Köln (DE)
(72) Erfinder: SODHA, Frank, 50670 Köln (DE); KÜHBACHER, Andreas, Dr., 50670 Köln (DE)
(74) Vertreter: Strehlke, Ingo Kurt

(57) **Zusammenfassung**

Die vorliegende Erfindung betrifft eine oxybutyninhaltige Zusammensetzung zur Verwendung bei der prophylaktischen bzw. therapeutischen Behandlung von insbesondere neurogenen Blasenfunktionsstörungen sowie deren Verwendungen, eine diesbezügliche Aufbewahrungs- bzw. Applikationsvorrichtung, eine Verpackungseinheit und ein Kit, welches insbesondere als Instillationssystem ausgebildet ist.

## Beschreibung

Die vorliegende Erfindung betrifft das medizinische Gebiet der Behandlung von insbesondere neurogenen Blasenfunktionsstörungen, welche insbesondere im Zusammenhang mit einer Detrusorhyperaktivität und/oder einer Detrusor-Sphinkter-Dyssynergie im Zusammenhang stehen und welche insbesondere infolge von Störungen oder Verletzungen des zentralen Nervensystems bzw. peripherer Nerven auftreten.

Insbesondere betrifft die vorliegende Erfindung eine oxybutyninhaltige Zusammensetzung zur Verwendung bei der prophylaktischen bzw. therapeutischen Behandlung von insbesondere neurogenen Blasenfunktionsstörungen, insbesondere von neurogenen Blasenentleerungsstörungen, vorzugsweise von mit einer Detrusorhyperaktivität bzw. einer Detrusor-Sphinkter-Dyssynergie einhergehenden neurogenen Blasenfunktionsstörungen, bevorzugt des Syndroms der insbesondere neurogen bedingten überaktiven Harnblase, wobei im Rahmen der vorliegenden Verwendung eine entsprechende Behandlungsdosis bestimmt bzw. festgelegt wird und wobei die Zusammensetzung nach der Erfindung mittels topischer Verabreichung, insbesondere mittels Instillation, in die Harnblase appliziert wird.

Die vorliegende Erfindung betrifft gleichermaßen auch die entsprechenden Verwendungen der erfindungsgemäßen oxybutyninhaltigen Zusammensetzung.

Darüber hinaus betrifft die vorliegende Erfindung auch eine Aufbewahrungs- bzw. Applikationsvorrichtung, insbesondere in Form einer Kolbenspritze, welche mit der erfindungsgemäßen oxybutyninhaltigen Zusammensetzung befüllt ist bzw. welche die Zusammensetzung nach der Erfindung aufweist.

Die vorliegende Erfindung betrifft gleichermaßen auch eine Verpackungseinheit, welche mindestens eine Aufbewahrungs- bzw. Applikationsvorrichtung nach der Erfindung aufweist, wobei die Aufbewahrungs- bzw. Applikationsvorrichtung in eine Verpackung eingebracht ist bzw. in einer Verpackung vorliegt.

Die vorliegende Erfindung betrifft in diesem Zusammenhang auch entsprechende Kits auf Basis der erfindungsgemäßen Aufbewahrungs- bzw. Applikationsvorrichtung bzw. der oxybutyninhaltigen Zusammensetzung nach der Erfindung.

Weiterhin betrifft die vorliegende Erfindung auch ein Verfahren zur Bestimmung bzw. Festlegung einer Behandlungsdosis, insbesondere einer tagesbezogenen Therapiedosis, von Oxybutynin bzw. Oxybutyninhydrochlorid als Wirkstoff bei der prophylaktischen bzw. therapeutischen Behandlung von insbesondere neurogenen Blasenfunktionsstörungen.

Neurogene Blasenfunktionsstörungen (nBFS) bzw. neurogene Blasenentleerungsstörungen stellen eine oftmals therapiebedürftige pathologische Situation der Harnblase und der an der Harnspeicherung und Harnausscheidung beteiligten anatomischen Strukturen da. Bei neurogenen Blasenfunktionsstörungen handelt es sich insbesondere um solche Dysfunktionen der Harnblase bzw. der relevanten anatomischen Strukturen, welche infolge einer Fehlfunktion oder Verletzung des Nervensystems auftreten, beispielsweise infolge von Rückenmarksverletzungen, Spina-bifida ("offener Rücken"), Diabetes, Multiple Sklerose, Schlaganfall, Morbus Parkinson oder dergleichen.

Im Allgemeinen wird als neurogene Blasenfunktionsstörung (nBFS) somit eine Störung der Harnspeicherung bzw. der Harnentleerung bezeichnet, welche durch neurologische Veränderungen bzw. Verletzungen im Bereich des Zentralnervensystems bzw. des Rückenmarks, im Bereich der für die Blasenfunktion relevanten Abschnitte des Gehirns oder im Bereich der Peripherie bzw. des peripheren Nervensystems verursacht wird bzw. hiermit im Zusammenhang steht, wobei eine normale nervöse bzw. nervale Signalübertragung zur Steuerung der Harnblase oftmals beeinträchtigt oder gestört bzw. sogar unterbrochen ist. Infolge der zugrundeliegenden neurologischen Schäden bzw. Fehlfunktionen ist oftmals die natürliche Funktion der Harnblase auch im Hinblick auf deren Funktionszustände, nämlich einer (Harn-)Füllphase zum einen und einer (Harn-)Entleerungs- bzw. Miktionsphase zum anderen, gestört. In diesem Zusammenhang kann der physiologische Ablauf von Blasenfüllung und Blasenentleerung sogar auf mehreren Ebenen beeinträchtigt bzw. unterbunden sein.

Infolge der neurologischen Fehlfunktion bzw. Störung liegt bei einer neurogenen Blasenfunktionsstörung (nBFS) oftmals eine Detrusorhyperaktivität und/oder Detrusor-Sphinkter-Dyssynergie vor. Damit geht häufig eine Störung des Zusammenspiels bzw. der Koordination der für die natürliche Blasenfunktion relevanten anatomischen Strukturen einher, was auch zu einer neurogen bedingten hyperaktiven Blase führen kann.

Der Detrusor (*Musculus detrusor vesicae* bzw. "Austreiber der Harnblase") als ein zentrales anatomisch-muskuläres Element der Harnblase stellt ein kräftiges Muskelsystem dar, welches die Harnblase umgibt bzw. einen Teil der Harnblase bildet bzw. welches die Harnblase formt, wobei es sich bei dem Detrusor um einen glatten Muskel handelt, welcher in der physiologischen Situation insbesondere bei der Miktion bzw. Blasenentleerung und somit der Harnaustreibung eine große Rolle spielt. In diesem Zusammenhang wird durch Signale des Parasympathikus eine Anspannung des Muskelsystems hervorgerufen, wodurch der Blaseninhalt unter Druck gesetzt wird. Zudem wird der Detrusor durch einen intrinsischen Nervenplexus beeinflusst, welcher in der Blasenwand liegt und deren muskulären Tonus dem Füllungszustand der Harnblase anpasst.

Demgegenüber liegt in der pathologischen Situation bei einer neurogenen Blasenfunktionsstörung oftmals eine Detrusorhyperaktivität vor, welche in diesem Zusammenhang auch als neurogene Detrusorhyperaktivität (*Neurogenic Detrusor Overactivity, NDO)* bezeichnet wird. Eine Detrusorhyperaktivität, welche mit einer relevanten neurologischen Grunderkrankung bzw. neurologischen Schädigung in Verbindung steht, wonach die Innervierung bzw. nervöse Steuerung des Detrusors unterbunden bzw. gestört ist, geht dabei insbesondere mit Pollakisurie, Nykturie sowie einer übermäßigen Drangsymptomatik sowie Harninkontinenz einher.

Bei einer Detrusor-Sphinkter-Dyssynergie (DSD) ist zudem das Zusammenwirken der bei der Blasenentleerung beteiligten anatomischen Strukturen gestört, wobei eine Detrusorüberaktivität oftmals einer spastischen Funktionsstörung der Beckenbodenmuskulatur bzw. des äußeren Blasenschließmuskels gegenübersteht, was zu einer Obstruktion des Blasenausgangs bei gleichzeitiger Miktionsanstrengung führt. Typisch für eine Detrusor-Sphinker-Dyssynergie ist dabei ein häufig unterbrochener Harnstrahl und Schwierigkeiten bei der Initiierung der Miktion. Darüber hinaus liegen oftmals auch Pollakisurie sowie eine Restharnbildung vor.

Eine neurogene bzw. neurologische Störung bzw. Fehlfunktion führt insgesamt häufig zu einer spastischen Harnblase, wobei es sich in diesem Zusammenhang auch derart verhalten kann, dass infolge der Fehlfunktion oder Verletzung des Nervensystems, wie zuvor angeführt, aufgrund der gestörten bzw. fehlenden nervösen Steuerung eine insgesamt überaktive Harnblase resultiert. In diesem Zusammenhang liegt oftmals eine hypersensitive, hypokapazitäre, instabile bzw. hypertone Harnblase, insbesondere einhergehend mit bzw. als Folge eines erhöhten Detrusordrucks, vor. Zudem ist neben einem erhöhten Detrusordruck oftmals der Detrusorkoeffizient, auch als *Compliance* der Harnblase bezeichnet, deutlich verringert, was zu entsprechenden Funktionseinschränkungen der Harnblase und zu diesbezüglichen Beschwerden führt.

Eine neurogene Blasenfunktionsstörung ist somit häufig auch mit hohen Detrusordrücken bzw. intravesikalen Drücken verbunden, wobei aufgrund der Hyperaktivität des Detrusors bzw. der zugrundeliegenden Detrusor-Sphinkter-Dyssynergie ein übermäßiger Detrusordruck bzw. ein übermäßiger Druck in der Harnblase aufgebaut wird. Dies stellt nicht zuletzt auch eine große Gefahr für den oberen Harntrakt dar. So gehen übermäßig hohe Detrusordrücke bzw. intravesikale Drücke oftmals mit einer Nierenschädigung einher bzw. rufen diese hervor. Bei entsprechend hohen Drücken in der Harnblase liegt häufig ein vesikoureteraler Reflux des Harns und somit eine retrograde Passage des Urins von der Harnblase zurück in die Harnleiter und mitunter auch in das Nierensystem vor, was zu einer entsprechenden Nierenschädigung führen kann. Weiterhin kann eine Abflussstörung von Urin aus dem Nierenbecken über die Harnleiter in die Blase resultieren, was gleichermaßen zu einer Nierenschädigung führen kann. Dabei können die vorgenannten Nierenschäden dauerhaft und irreversibel sein.

Ein gestörter Urinabfluss durch ureterale Obstruktion führt zur Druckübertragung auf das Nierengewebe. Es kommt zur Durchblutungsstörung in der Niere mit lokaler Ischämie und in der Folge zur Inflammation des Nierengewebes und Fibrosierung und schlussendlich zu einem Schaden des renalen Filtationsapparates (Tubulusschaden) und Reduktion der Filtrationsleistung. Gleiche Vorgänge gelten für einen Reflux, der zusätzlich noch direkt zu einer Nierenvernarbung und zu einem Gewebeuntergang durch Infekte führen kann.

Dabei verhält es sich bei einer neurogenen Blasenfunktionsstörung der vorgenannten Art auch derart, dass aufgrund der zugrundliegenden Fehlfunktionen hohe Detrusordrücke von mehr als 40 cm H₂O (40 Zentimeter Wassersäule) vorliegen, was im Hinblick auf die Ausbildung eines unerwünschten Refluxes in die Niere und damit einhergehenden Nierenschäden als kritisch zu beurteilen ist.

Als weitere Komplikation, welche im Zusammenhang mit einer neurogenen Blasenfunktionsstörung stehen kann, resultiert, wie zuvor angeführt, ein oftmals verringerter Detrusorkoeffizient bzw. eine verringerte *Compliance* der Harnblase, wobei der Detrusorkoeffizient bzw. die *Compliance* ein Maß zur Beurteilung der Blasendehnbarkeit bzw. des Muskeltonus der Blasenwand ist. Dementsprechend liegt häufig auch eine unzureichende Blasendehnbarkeit bzw. ein erhöhter Muskeltonus der Blasenwand vor, was in entsprechender Weise mit einer Verschlechterung der Blasenfunktion und der Gefahr etwaiger Folgeschäden einhergeht.

Insbesondere liegt infolge der neurogenen Grunderkrankung bzw. Schädigung oftmals eine überaktive Harnblase bzw. das Syndrom der überaktiven Harnblase vor. Infolge der hohen Drücke innerhalb der Harnblase kann es auch zu anatomischen bzw. pathologischen Veränderungen der Harnblase selbst kommen (wie Divertikelbildung, Muskelhypertrophie, fibrotischer Blasenschrumpfung), einhergehend mit einer weiterführenden Verschlechterung der Blasenfunktion.

Der mit der Fehl- bzw. Dysfunktion der Harnblase insgesamt einhergehende Leidensdruck von Patienten ist dabei mitunter sehr groß, zumal die aufgrund der Schwere der Grunderkrankung oftmals schon eingeschränkte Lebensqualität durch die mit der Dysfunktion einhergehenden Beschwerden zusätzlich verschlechtert wird. Beispielsweise kann bei Querschnittpatienten das potentiell lebensbedrohliche Krankheitsbild der autonomen Dysregulation resultieren.

Vor dem Hintergrund der obigen Ausführungen besteht somit im Stand der Technik ein großer Bedarf an der Bereitstellung therapeutischer Konzepte, welche bei Vorliegen einer neurogenen Blasenfunktionsstörung zu einer Verbesserung der Blasenfunktion bzw. zu einer Verringerung der Symptome führen, und zwar sowohl was die Probleme beim Harnlassen als auch die vorliegenden hohen Drücke in der Harnblase anbelangt. Dabei besteht ein wesentliches therapeutisches Ziel neben der Verbesserung der Miktion auch in der Verringerung des Detrusordrucks in der Harnblase, um hierdurch etwaige Nierenschäden oder dergleichen zu vermeiden.

Neben konservativen Maßnahmen, wie z.B. gezieltes Blasentraining oder dergleichen, kommen auch pharmakotherapeutische Maßnahmen zur Behandlung neurogener Blasenfunktionsstörungen zum Einsatz. Beispielsweise kann auf Basis minimalinvasiver Verfahren eine Injektion mit Botulinumtoxin in die Harnblasenwand durchgeführt werden, um hierdurch die Aktivität und Kontraktilität des Detrusors zu vermindern. Die Verabreichung ist jedoch relativ aufwendig und nur durch einen Arzt durchzuführen, da die Wirksubstanz relativ aufwendig in die Blasenwandung injiziert werden muss, was aber zu Verletzungen bzw. Entzündungen führen kann. Weiterhin kommen auch minimalinvasive Verfahren in Form von sakraler Neuromodulation zum Einsatz, bei welcher im oberen Gesäßbereich ein Schrittmacher implantiert wird, welcher über eine Elektrode schwache elektrische Impulse an die Sakralnerven abgibt. Der diesbezügliche Einsatzbereich ist jedoch im Hinblick auf die zugrundeliegenden Grunderkrankungen beschränkt. Zudem handelt es sich hierbei um einen operativen Eingriff, welcher mit weiteren Risiken einhergeht.

Weiterhin sind im Stand der Technik pharmakologische Therapien bekannt, bei denen auch Anticholinergika zum Einsatz kommen. In diesem Zusammenhang wird insbesondere auf eine Verabreichung von Oxybutynin bzw. Oxybutyninhydrochlorid, Trospiumchlorid, Propiverin, Tolterodin oder dergleichen abgestellt. Dabei besteht im Stand der Technik ein Fokus auf einer systemischen bzw. oralen Applikation bzw. Verabreichung dieser Wirksubstanzen. Die systemische Verabreichung kann jedoch im verstärkten Maße mit systemischen Nebenwirkungen einhergehen, wie Restharnbildung, Obstipation, Akkomodationsstörungen, Mundtrockenheit, Tachykardie, Herzrhythmusstörungen und dergleichen. Darüber hinaus ist die Menge bzw. Dosis an Wirksubstanz am Wirkort, nämlich der Harnblase, mitunter nicht optimal einzustellen.

Darüber hinaus ist eine systemische Verabreichung mit peroraler Applikation von Wirksubstanzen in Form von Anticholinergika insofern nachteilig, als ein Teil der verabreichten und über den Gastrointestinaltrakt aufgenommenen Wirksubstanz bei der ersten Passage bzw. in der Leber verstoffwechselt wird (sogenannter *First pass-Metabolismus*), wobei mitunter nichtwirksame Metaboliten entstehen, so dass die Bioverfügbarkeit am Wirkort nochmals geringer ausfällt bzw. die Wirkstoffmenge am Wirkort nur schwer einzustellen bzw. vorzugeben ist. Folglich können Patienten mit Darreichungsformen zur peroralen Applikation mitunter nicht hinreichend eingestellt werden.

Die WO 2012/154779 A1 bzw. die zu derselben Patentfamilie gehörende US 2012/0289564 A1 betrifft die Verwendung einer Kombination von Oxybutynin und einer die Bildung von Speichel anregenden Substanz zur Behandlung der überaktiven Blase, wobei diesbezüglich auf eine perorale Applikation fokussiert wird.

Darüber hinaus können Anticholinergika topisch appliziert werden, beispielsweise mittels eines transdermalen Pflasters oder dergleichen. Auch bei dieser systemischen Applikation können trotz Umgehung bzw. Reduktion des *First pass-*Metabolismus systemische Nebenwirkungen auftreten. Zudem besteht auch hier das Problem einer vorzeitigen Verstoffwechselung mit der Unbestimmtheit der am Wirkort vorliegenden Wirkstoffmenge.

Darüber hinaus können Anticholinergika, wie Oxybutynin bzw. Oxybutyninhydrochlorid, topisch in den Urogenitalbereich, insbesondere in die Harnblase, appliziert bzw. instilliert werden, typischerweise über einen Katheter oder dergleichen.

Oxybutynin bzw. Oxybutyninhydrochlorid gehört insbesondere zu der Gruppe der sogenannten Parasympatholytika. Oxybutynin bzw. Oxybutyninhydrochlorid weist einen direkten spasmolytischen Effekt auf glatte Muskulatur auf, wobei es anticholinerg wirkt, indem es die Acetylcholinwirkung an der glatten Muskulatur hemmt.

Oxybutynin bzw. Oxybutyninhydrochlorid wirkt dabei insbesondere als kompetitiver Antagonist von Acetylcholin auf postganglionäre Muskarinrezeptoren, was zu einer Abnahme der Detrusorhyperaktivität und somit zur Entspannung bzw. krampflösenden Wirkung im Hinblick auf die glatte Blasenmuskulatur führt. Insbesondere wird die Detrusor- bzw. Blasenkontraktion gehemmt, wobei auch Spasmen gelockert werden. Zudem führt die Verabreichung von Oxybutynin bzw. Oxybutyninhydrochlorid zu einer Vergrößerung des Blasenvolumens und zu einer Verringerung von Kontraktionen, wobei auch der Drang zum Harnlassen bei neurogenen Blasenfunktionsstörungen verringert bzw. verzögert wird. Weiterhin kann durch den Einsatz von Oxybutynin bzw. Oxybutyninhydrochlorid auch der Detrusordruck bzw. der intravesikale Blasendruck verringert werden, was auch zu einer Entlastung der oberen Harnwege führt. Darüber hinaus weist Oxybutynin bzw. Oxybutyninhydrochlorid auch Eigenschaften eines Lokalanästhetikums auf.

Im Rahmen der im Stand der Technik vorgesehenen Instillation bzw. topischen Applikation in die Harnblase hat es sich aber oftmals als schwierig bzw. problematisch erwiesen, eine diesbezüglich optimierte Therapie im Hinblick auf eine entsprechende Dosisfindung der Wirksubstanz zu gewährleisten, so dass im Stand der Technik eine entsprechend optimierte Einstellung des Patienten mitunter nur begrenzt möglich ist, einhergehend mit einem nicht optimalen Therapieerfolg.

Im Stand der Technik wird bei der Dosisfindung oftmals ausschließlich von subjektiven Kriterien ausgegangen, und zwar ohne Berücksichtigung weiterführender Zielparameter, wobei zudem oftmals ein mehr oder weniger starres und nicht adaptiertes Therapieregime herangezogen wird. Insbesondere wird im Stand der Technik maßgeblich oftmals nur von der Grunderkrankung als solcher ausgegangen, wobei die herangezogenen bzw. eingesetzten Wirksubstanzmengen bzw. -dosen sozusagen pauschal und ohne weiterführende Adaptierung bzw. Anpassung an die konkret vorliegende pathologische Blasensituation bzw. -fehlfunktion erfolgt.

Insbesondere fehlt es im Stand der Technik an einem weiterführenden Therapiekonzept unter Anpassung bzw. Auswahl einer zu verabreichenden Wirkstoffmenge unter Einbezug objektiver Kenngrößen bzw. Parameter, so dass diesbezüglich im Stand der Technik eine optimale Dosisfindung zur Gewährleistung eines optimalen Therapieerfolgs in dieser Form nicht gegeben ist.

Auch von daher besteht im Stand der Technik ein nochmals weiterführender Bedarf an der Bereitstellung entsprechender Therapie- und Dosisfindungskonzepte, welche zu einer verbesserten Behandlung von Patienten mit neurogenen Blasenfunktionsstörungen mit möglichst weitreichender Verbesserung der Blasenfunktion führen, um hierdurch zum einen die physiologische Funktion der Harnblase so weit wie möglich zu verbessern (und zwar auch unter dem Aspekt der Vermeidung von Folgeschäden wie eine Schädigung der Nieren oder dergleichen) und zum anderen den Leidensdruck der Patienten zu verringern.

Vor diesem Hintergrund besteht somit eine Aufgabe der vorliegenden Erfindung darin, ein gegenüber dem Stand der Technik verbessertes Therapie- bzw. Dosisfindungskonzept im Hinblick auf eine oxybutyninhaltige Zusammensetzung zur prophylaktischen bzw. therapeutischen Behandlung von insbesondere neurogenen Blasenfunktionsstörungen, insbesondere von neurogenen Blasenentleerungsstörungen, vorzugsweise von mit einer Detrusorhyperaktivität bzw. einer Detrusor-Sphinkter-Dyssynergie einhergehenden neurogenen Blasenfunktionsstörungen bzw. des Syndroms der insbesondere neurogen bedingten überaktiven Harnblase, bereitzustellen, wobei die zuvor geschilderten Nachteile des Standes der Technik zumindest weitgehend vermieden oder aber wenigstens abgeschwächt werden sollen.

Insbesondere ist eine Aufgabe der vorliegenden Erfindung darin zu sehen, eine diesbezügliche oxybutyninhaltige Zusammensetzung zur Verwendung bei der prophylaktischen bzw. therapeutischen Behandlung von insbesondere neurogenen Blasenfunktionsstörungen bereitzustellen, wobei die Zusammensetzung zur Gewährleistung eines optimalen Therapieerfolgs mit diesbezüglich gezielt eingestellten Mengen bzw. Dosen der Wirksubstanz in Form von Oxybutynin bzw. Oxybutyninhydrochlorid mittels topischer Verabreichung, insbesondere mittels Instillation, in die Harnblase appliziert werden soll, so dass auf dieser Basis eine verbesserte Einstellung der Blasenfunktion gewährleistet wird.

Insbesondere soll im Rahmen einer weiteren Aufgabe der vorliegenden Erfindung ein entsprechendes Therapieregime unter Auffindung optimierter Dosen an Wirksubstanz für eine oxybutyninhaltige Zusammensetzung zur Verwendung bei der prophylaktischen bzw. therapeutischen Behandlung von insbesondere neurogenen Blasenfunktionsstörungen bereitgestellt werden, und zwar insbesondere vor dem Hintergrund einer optimierten Einstellung der Harnblasenfunktion und insbesondere zu Zwecken eines weiterführenden Schutzes des oberen Harntrakts bzw. der Nieren. Zudem soll das bereitgestellte Therapieregime zu einer nachhaltigen Verbesserung auch der Kontinenz bei gleichzeitiger Verringerung von Nebenwirkungen führen, und dies auch vor dem Hintergrund einer diesbezüglichen Langzeittherapie.

Zudem soll im Rahmen einer weiteren Aufgabe der vorliegenden Erfindung eine entsprechende oxybutyninhaltige Zusammensetzung in applikations- bzw. anwendungsfertiger Form bereitgestellt werden, wobei erfindungsgemäß eine sichere Anwendung bzw. Applikation der Zusammensetzung gewährleistet werden soll, und dies nicht zuletzt auch im Hinblick auf die Bereitstellung einer diesbezüglichen Zusammensetzung mit hoher Keimfreiheit bzw. hoher Sterilität sowie hoher (Lager-)Stabilität.

Zur Lösung der zuvor geschilderten Aufgabe schlägt die vorliegende Erfindung somit - gemäß einem **ersten** Aspekt der vorliegenden Erfindung - eine oxybutyninhaltige Zusammensetzung zur Verwendung bei der prophylaktischen bzw. therapeutischen Behandlung von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, gemäß dem diesbezüglichen, die erfindungsgemäße Zusammensetzung betreffenden unabhängigen Anspruch vor; jeweils vorteilhafte Weiterbildungen und Ausgestaltungen dieses Erfindungsaspekts sind Gegenstand der die Zusammensetzung nach der Erfindung betreffenden Neben- und Unteransprüche.

Weiterhin betrifft die vorliegende Erfindung - gemäß einem **zweiten** Aspekt der vorliegenden Erfindung - auch die Verwendung der Zusammensetzung nach der Erfindung.

Zudem betrifft die vorliegende Erfindung - gemäß einem **dritten** Aspekt der vorliegenden Erfindung - eine die erfindungsgemäße Zusammensetzung enthaltende Aufbewahrungs- bzw. Applikationsvorrichtung, wie sie in dem diesbezüglichen unabhängigen Anspruch definiert ist.

Darüber hinaus betrifft die vorliegende Erfindung - gemäß einem **vierten** Aspekt der vorliegenden Erfindung - auch eine Verpackungseinheit, welche die Aufbewahrungs- bzw. Applikationsvorrichtung nach der Erfindung enthält, gemäß dem diesbezüglichen, die Verpackungseinheit nach der Erfindung betreffenden unabhängigen Anspruch.

Die vorliegende Erfindung betrifft zudem - gemäß einem **fünften** Aspekt der vorliegenden Erfindung - auch ein Kit, insbesondere ein Applikations- bzw. Instillationssystem, wie es in dem das Kit betreffenden unabhängigen Anspruch definiert ist.

Schließlich betrifft die vorliegende Erfindung - gemäß einem **sechsten** Aspekt der vorliegenden Erfindung - auch ein Verfahren zur Bestimmung und/oder Festlegung einer Behandlungsdosis von Oxybutynin bzw. Oxybutyninhydrochlorid als Wirkstoff bei der prophylaktischen bzw. therapeutischen Behandlung von insbesondere neurogenen Blasenfunktionsstörungen gemäß dem das erfindungsgemäße Verfahren betreffenden unabhängigen Anspruch.

Es versteht sich von selbst, dass Ausgestaltungen, Ausführungsformen, Vorteile und dergleichen, welche nachfolgend zu Zwecken der Vermeidung von Wiederholungen zur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Darüber hinaus verhält es sich im Hinblick auf die nachfolgende Beschreibung der vorliegenden Erfindung auch derart, dass die jeweils im Zusammenhang mit den speziellen Ausgestaltungen, Ausführungsformen, Vorteilen, Beispielen oder dergleichen angeführten Merkmale der vorliegenden Erfindung auch in deren Kombination als offenbart gelten. Somit gelten vorliegend auch übergeordnete Kombinationen einzelner oder mehrerer Merkmale, welche für jeweilige Ausgestaltungen, Ausführungsformen, Anwendungsbeispiele oder dergleichen angeführt sind, als offenbart.

Bei allen nachstehend genannten relativen bzw. prozentualen gewichtsbezogenen Angaben, insbesondere Mengen- bzw. Konzentrationsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, zu 100 % bzw. 100 Gew.-% ergänzen bzw. addieren; dies versteht sich aber für den Fachmann von selbst.

Weiterhin gilt, dass der Fachmann - anwendungsbezogen oder einzelfallbedingt - von den nachfolgend angeführten Konzentrations-, Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren und andernfalls mit dem Fachmann auf diesem Gebiet an sich geläufigen Bestimmungs- bzw. Messmethoden ermittelt bzw. bestimmt werden können. Sofern nicht anders angegeben, werden die zugrundeliegenden Werte bzw. Parameter unter Standardbedingungen (d. h. insbesondere bei einer Temperatur von 20 °C und/oder bei einem Druck von 1.013,25 hPa bzw. 1,01325 bar) ermittelt.

Der Begriff des Arzneimittels bzw. Medikaments (synonym auch als "Pharmazeutikum" bezeichnet), wie er im Rahmen der vorliegenden Erfindung verwendet wird, ist vorliegend sehr umfänglich bzw. breit zu verstehen und umfasst nicht nur Arzneimittel bzw. Pharmazeutika als solche (d. h. in arzneimittelrechtlicher Hinsicht), sondern weiterhin auch sogenannte Medizinprodukte und darüber hinaus aber auch Homöopathika und Nahrungsergänzungsmittel sowie Kosmetika und Gebrauchsgegenstände. Mit anderen Worten kann also die erfindungsgemäße Zusammensetzung in Form einer pharmazeutischen Zusammensetzung, eines Arzneimittels (Pharmazeutikums), Medizinprodukts, Homöopathikums, Nahrungsergänzungsmittels, Kosmetikums oder in Form eines Gebrauchsgegenstands vorliegen.

Dies vorausgeschickt wird im Folgenden die vorliegende Erfindung im Detail erläutert:
Gegenstand der vorliegenden Erfindung - gemäß einem **ersten** Aspekt der vorliegenden Erfindung - ist somit die oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird,
wobei die Zusammensetzung das Oxybutynin bevorzugt in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
   - Oxybutynin, bevorzugt Oxybutyninhydrochlorid (Oxybutynin-HCl), in einer Konzentration von (1 ± 0,5) mg/ml und
   - mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,5) mg/ml
enthält,

wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion.

Im Rahmen der vorliegenden Erfindung wird somit auf Basis der erfindungsgemäßen Zusammensetzung mit der diesbezüglichen speziellen Verwendung ein spezielles Therapie- bzw. Dosisfindungskonzept bzw. ein spezielles Therapieregime bereitgestellt, anhand dessen die der Applikation der oxybutyninhaltigen Zusammensetzung im Rahmen der prophylaktischen bzw. therapeutischen Behandlung zugrundeliegende Wirkstoffmenge bzw. Dosis an Oxybutynin bzw. Oxybutyninhydrochlorid zur Behandlung von insbesondere neurogenen Blasenfunktionsstörungen gezielt und unter Einbezug objektiver Zielparameter bzw. Kenngrößen eingestellt bzw. vorgegeben wird. Auf dieser Basis kann im Rahmen der vorliegenden Erfindung eine Therapieoptimierung im Hinblick auf die zu behandelnden insbesondere neurogenen Blasenfunktionsstörungen unter weiterführender Verbesserung der Blasenfunktion bereitgestellt werden, und dies unter gleichzeitiger Minimierung bzw. Verringerung von Nebenwirkungen.

Die vorliegende Erfindung fokussiert dabei im Hinblick auf die Dosisfindung maßgeblich auch auf objektiv bestimmbare urodynamische Kenngrößen bzw. Messgrößen des zu behandelnden Patienten, und zwar insbesondere was den Detrusordruck, den Detrusorkoeffizienten bzw. die *Compliance* sowie den intravesikalen Druck anbelangt, welche im Rahmen der erfindungsgemäßen Konzeption ziel- und zweckgerichtet als Zielparameter herangezogen werden und durch entsprechende Bestimmung und Festlegung der einzusetzenden Mengen bzw. Dosen an Wirksubstanz gezielt eingestellt werden.

Insbesondere handelt es sich bei den herangezogenen urodynamischen Kenngrößen bzw. Messgrößen um objektive Zielparameter, welche sozusagen als Surrogatparameter schnell und einfach in objektiver Weise erfasst bzw. bestimmt werden können, beispielsweise unter Heranziehung entsprechender urodynamischer Mess- bzw. Bestimmungsverfahren. Dabei können die zuvor genannten urodynamischen Kenngrößen anhand urodynamischer Messungen bestimmt werden und auf dieser Basis die Dosis der Wirksubstanz in Form von Oxybutynin bzw. Oxybutyninhydrochlorid gezielt vorgegeben bzw. eingestellt werden, um hierdurch eine Verbesserung der entsprechenden Parameter zu erhalten, nämlich insbesondere im Hinblick auf eine Verringerung des Detrusordrucks sowie des intravesikalen Drucks einerseits bzw. eine Erhöhung des Detrusorkoeffizienten bzw. der *Compliance* der Harnblase.

Bei den objektiven Parametern insbesondere in Form des Detrusordrucks, Detrusorkoeffizienten bzw. der *Compliance* und des intravesikalen Drucks handelt es sich somit um physikalisch-physiologische Parameter, welche auf Basis urodynamischer Messungen bestimmt werden können.

Bei den vorgenannten urodynamischen Kenngrößen handelt es sich, wie zuvor angeführt, gleichermaßen um Zielparameter, welche im Rahmen der vorliegenden Erfindung im Hinblick auf einen therapeutischen Effekt durch optimierte und an die zugrundeliegenden Zielparameter ausgerichtete Dosis der Wirksubstanz Oxybutynin bzw. Oxybutyninhydrochlorid in entsprechender Weise zur Bereitstellung eines Therapieerfolgs therapeutisch-medikamentös eingestellt bzw. verbessert werden.

Dabei kann im Rahmen der vorliegenden Erfindung auch eine weiterführende Dosisanpassung im Verlauf einer (Langzeit-)Therapie erfolgen, und zwar durch regelmäßige Kontrolle bzw. Erfassung der urodynamischen Kenngrößen mit entsprechender Einstellung dieser Parameter auf Basis einer entsprechenden Anpassung der Wirkstoffmenge.

Im Rahmen der vorliegenden Erfindung wird somit ein an entsprechende Zielparameter, wie zuvor definiert, adaptiertes Therapieregime zu Zwecken einer optimierten Einstellung der Harnblasenfunktion bei insbesondere neurogenen Blasenfunktionsstörungen bereitgestellt.

In diesem Zusammenhang finden maßgeblich allenfalls subjektiv bestimmbare und messtechnisch weniger objektiv zu erfassende Parameter, wie Harnblasenkapazität, Blasenfüllungssensitivität sowie Gefühl des ersten Harndrangs und dergleichen, bei der Bestimmung der erfindungsgemäß einzusetzenden Behandlungsdosis bzw. der tagesbezogenen Therapiedosis eine lediglich optionale und nachgeordnete Berücksichtigung, wie nachfolgend noch ausgeführt, wobei diese Parameter dann sogar zu einer weiterführenden Präzisierung der sozusagen vorgeschalteten objektiven Parameter führen, so dass auch von daher eine weiterführende Objektivierung hinsichtlich der heranzuziehenden Behandlungsdosen bzw. der zugrundeliegenden Dosisfindung vorliegt.

Erfindungsgemäß wird somit eine objektiv basierte Therapieoptimierung ausgehend von objektiven Messparametern bzw. urodynamischen Kenngrößen bereitgestellt, wobei die zugrundeliegenden Kenngrößen, wie Detrusordruck, Detrusorkoeffizient bzw. *Compliance* sowie intravesikaler Druck ausgehend von einer einheitlichen Urodynamik bestimmt werden können, so dass erfindungsgemäß im Gegensatz zum Stand der Technik eine im Hinblick auf eine Therapieoptimierung definierte und nicht beliebige Dosisfindung vorliegt, was zu einer weiterführenden Wirkoptimierung mit verringerten Nebenwirkungen des eingesetzten Wirkstoffs in Form von Oxybutynin bzw. Oxybutyninhydrochlorid führt.

Erfindungsgemäß wird somit sozusagen eine objektivierte Therapieoptimierung ausgehend von objektiven Messparametern bereitgestellt, und dies auf Basis eines befundadaptierten und patientenindividualisierten Ansatzes, ausgehend von einer definierten Urodynamik.

Erfindungsgemäß wird somit insgesamt eine objektivierte Therapie- bzw. Dosisfindung maßgeblich bzw. vorrangig anhand physiologisch-physikalischer Kenngrößen bzw. Messdaten bereitgestellt, welche durch weitere Kenngrößen gegebenenfalls ergänzt werden können.

Im Rahmen der vorliegenden Erfindung können folglich insgesamt optimierte Behandlungsdosen festgelegt und bestimmt werden, und zwar maßgeblich auf Basis objektiver physiologisch-physikalischer Kenngrößen und in weiterführender Abstimmung mit weiteren Kenngrößen, was erstmals eine optimierte und objektivierte Therapiefindung ermöglicht.

Was die vorliegende Erfindung weiterhin anbelangt, so führt die oxybutyninhaltige Zusammensetzung nach der Erfindung im Rahmen ihrer Verwendung bei der Behandlung von insbesondere neurogenen Blasenfunktionsstörungen unter Einbezug der diesbezüglich heranzuziehenden Behandlungsdosis zu einer nachhaltigen Verbesserung der pathologischen Blasensituation, und zwar auch was die gezielte Verringerung des Detrusor- bzw. Harnblasendrucks sowie die Erhöhung der *Compliance* anbelangt. Folglich kann erfindungsgemäß eine verbesserte Situation hinsichtlich der natürlichen Harnblasenfunktion erreicht werden, und zwar auch im Hinblick auf einen Schutz des oberen Harntraktes bzw. der Nieren, der Erzielung einer verbesserten Kontinenz sowie der Verringerung von Nebenwirkungen. Insbesondere kann auch eine weiterführende Reduktion der Miktionsfrequenz sowie der Dranginkontinenzepisoden bei weiterführender Verbesserung der Harnblasenkapazität, Harnblasenfüllungssensitivität sowie Zeitpunkt des Gefühls des ersten Harndrangs erreicht werden.

Zudem wird im Rahmen der vorliegenden Erfindung insgesamt auch eine applikations- bzw. anwendungsfertige Zusammensetzung bereitgestellt, welche im Rahmen des erfindungsgemäßen Konzepts eine hohe Anwendungssicherheit aufweist.

Was die erfindungsgemäß angeführte Behandlungsdosis bzw. die tagesbezogene Therapiedosis anbelangt, so bezieht sich diese insbesondere auf die Applikation der oxybutyninhaltigen Zusammensetzung nach der Erfindung im Rahmen der therapeutischen Behandlung der zugrundeliegenden insbesondere neurogenen Blasenfunktionsstörungen mit den damit einhergehenden Mengen bzw. Dosen der Wirksubstanz in Form von Oxybutynin, bevorzugt Oxybutyninhydrochlorid.

Was das erfindungsgemäß als Wirksubstanz eingesetzte Oxybutynin bzw. Oxybutyninhydrochlorid anbelangt, so handelt es sich hierbei, wie zuvor angeführt, um ein Anticholinergikum, welches krampflösend bzw. spasmolytisch auf die glatte Blasenmuskulatur wirkt, wie zuvor angeführt. Oxybutynin ist der internationale Freiname für 4-Diethylaminobut-2-inyl-2-cyclohexyl-2-hydroxy-2-phenylethanoat (IUPAC) bzw. α-Cyclohexyl-α-hydroxyphenylessigsäure-4-(diethylamino)-2-butinylester. Erfindungsgemäß wird insbesondere das Hydrochlorid (Oxybutyninhydrochlorid, Oxybutynin-HCl) verwendet. Für diesbezüglich weiterführende Informationen zu Oxybutynin bzw. Oxybutyninhydrochlorid kann auf die Ausführungen in RÖMPP Chemielexikon, 10. Auflage, Band 4, 1998, Georg Thieme Verlag, Stuttgart / New York, Seite 3081, Stichwort: "Oxybutynin", sowie auf die dort referierte Literatur verwiesen werden, wobei der diesbezügliche Gesamtoffenbarungsgehalt hiermit durch Bezugnahme vollumfänglich eingeschlossen ist.

Im Rahmen der vorliegenden Erfindung werden die Begriffe "Harnblase" einerseits und "Blase" andererseits synonym verwendet.

Wie zuvor angeführt, wird die Behandlungsdosis bzw. die tagesbezogene Therapiedosis auch unter Einbezug urodynamischer Kenngrößen, wie insbesondere unter (i) angeführt, bestimmt bzw. festgelegt. Zur diesbezüglichen Bestimmung der Kenngrößen kann auch auf Folgendes verwiesen werden:
Sowohl qualitative als auch quantitative Informationen auch zu den erfindungsgemäß angeführten Kenngrößen, wie zur Detrusorfunktion, lassen sich beispielsweise über eine Blasendruckmessung bzw. Zystometrie gewinnen. Die Registrierung des intravesikalen Druckes (pᵥₑₛ) unter kontinuierlicher Blasenfüllung (Füllungsphase) bzw. während der Miktion (Miktionsphase) lässt dabei insbesondere auch Aussagen zur Blasenmotorik, zu den elastischen Eigenschaften (Dehnbarkeit) des Detrusors und zu neurophysiologischen Störformen der Blaseninnervation zu. Zudem werden indirekt der Blasenauslasswiderstand während der Miktion sowie die extero- und propriozeptiven Kenngrößen (Sensitivität und Harndrang) der Blasensensorik erfasst.

Zur Verbesserung von Standardisierung und Reproduzierbarkeit sowie zur interindividuellen Vergleichbarkeit der Ergebnisse können sämtliche Messgrößen gemäß den Empfehlungen des Standardisierungskomitees der International Continence Society (ICS) bestimmt und angegeben werden, einschließlich der technischen Ausstattungen und der Messmethode.

Als standardisierte Messeinheit bei der Urodynamik wird der Druck stets in Zentimeter Wassersäule ([cm H₂O]) angegeben. Unter Berücksichtigung der SI-Einheiten entspricht 1 cm H₂O = 98,07 Pascal. Entsprechend dem Funktionszustand der Blase können in der Füllungsphase folgende detrusorspezifischen Parameter erfasst bzw. errechnet werden:
- Intravesikaler Druck (pᵥₑₛ [cm H₂O]);
- Maximale Blasenkapazität;
- Detrusorkoeffizient (*Compliance* C [ml H₂O]);
- Blasenfüllungssensitivität;
- Gefühl des ersten Harndrangs ([ml]).

Bei der Bestimmung der Blasenkapazität kann im Allgemeinen zwischen zystometrischer und funktioneller Blasenkapazität unterschieden werden. Die zystometrische Blasenkapazität entspricht dem Blasenfüllungsvolumen, bei dem ein Patient ein nicht weiter unterdrückbares Harndranggefühl wahrnimmt. Die funktionelle Blasenkapazität errechnet sich aus der Differenz zwischen zystometrischer Kapazität und verbliebenem Restharn.

Der Detrusorkoeffizient (*Compliance*) ist ein Maß zur Beurteilung der Blasendehnbarkeit bzw. des Muskeltonus der Blasenwand. Errechnet wird die *Compliance* (C [ΔV/Δp]) aus dem Quotienten der Füllungsvolumenzunahme (ΔV = ml) und dem korrelierenden intravesikalen Druckanstieg (Δp [cm H₂O]). Zwei wesentliche Punkte spielen bei der Berechnung der Compliance eine wesentliche Rolle, und zwar der Detrusordruck zu Beginn der Blasenfüllung mit dem korrespondierenden Blasenvolumen (in der Regel 0 ml) zum einen und der Detrusordruck (und das korrespondierende Blasenvolumen) bei Erreichen der zystometrischen Blasenkapazität oder vor einer unwillkürlichen Detrusorkontraktion mit/ohne Urinverlust zum anderen. Neben dem intravesikalen Druck sollte stets der Abdominal- bzw. Rektaldruck (p_{abd} [cm H₂O]) simultan registriert werden, da ansonsten die Beurteilung einer Detrusorstabilität und einer Harninkontinenz gegebenenfalls nur unzureichend möglich ist. Da zystometrische Untersuchungen meist als Kombinationsuntersuchungen ausgelegt sind, z.B. Druckflussmessungen mit oder ohne Beckenbodenelektromyographie, können die harnflussspezifischen Messdaten miterfasst werden. Aus der Differenz der zystometrischen Blasenkapazität und dem Miktionsvolumen errechnet sich das Restharnvolumen. Das simultan aufgezeichnete Beckenboden-EMG gibt Aufschluss über die Wechselwirkungen zwischen Detrusor und Harnröhrensphinkter und kann als direkter Parameter für die Beurteilung des synergenen Verhaltens dieser beiden Muskelgruppen herangezogen werden.

Im Hinblick auf die Füllungsphase kann es sich im Allgemeinen derart verhalten, dass die Blasenfüllungsgeschwindigkeit physiologisch oder unphysiologisch sein kann. Die physiologische Füllgeschwindigkeit wird berechnet nach der Formel: Körpergewicht in kg dividiert durch 4 ausgedrückt als ml/min. Als nichtphysiologische Füllgeschwindigkeit werden Füllungsraten größer als diese bezeichnet. Im Allgemeinen sollte die Füllungsgeschwindigkeit bei Routineuntersuchungen 30 ml/min nicht überschreiten, da es ansonsten zur Provokation einer Detrusorkontraktion kommen kann.

Im Rahmen der gesamten urodynamischen Funktionsdiagnostik können Informationen, wie Sensitivität, erster Harndrang usw. bestimmt werden. Während der Blasenfüllungsphase können gegebenenfalls auch in Abhängigkeit von der Symptomatik zudem Provokationstests (Husten, Aufstehen, Beklopfen der Bauchdecke, Schnellfüllung, Eiswassertest) durchgeführt werden, um eine mögliche Fehlfunktion (Dranginkontinenz, Giggle-Inkontinenz, Belastungsinkontinenz) nachzuweisen. Gegebenenfalls sollten die gleichen Tests bei unterschiedlichen Füllungszuständen wiederholt werden. Insbesondere wird die Blasenfüllung bis zum Erreichen der zystometrischen Blasenkapazität (starkes, imperatives Harndranggefühl) fortgeführt, bevor die Entleerungsphase eingeleitet wird.

In Bezug auf einen Normalbefund erfährt die Blasenwandmuskulatur ausgehend von einem intravesikalen und abdominellen Ruhedruck zwischen 6 cm H₂O und 15 cm H₂O mit Einsetzen der Blasenfüllung eine kontinuierliche Dehnung.

Dabei wird durch die myoelastischen Eigenschaften des Detrusors der intravesikale Druck trotz zunehmender Blasenfüllung kompensiert, so dass mit Volumenzunahme der vesikale Druck nur sehr langsam ansteigt. Im Normalfall, d.h. bei unbeeinträchtigter Blasendehnbarkeit, liegt die Maßzahl für die *Compliance* im Allgemeinen über 25 ml/cm H₂O (z.B. 100 ml Volumenzunahme / 4 cm H₂O Druckänderung = 25 ml/cm H₂O). Bestimmt wird der *Compliance-*Wert zwischen Füllungsbeginn und erstem Harndrang. Liegt der Blasendehnungskoeffizient unterhalb der Normgrenze von 25 ml/cm H₂O, so liegt eine eingeschränkte Blasendehnbarkeit vor, wie es bei einer neurogenen Blasenstörung, oder einer *low compliance bladder* der Fall sein kann.

Mit zunehmender Füllung (30 ml/min) geben die Patienten im Allgemeinen unter Normalbefund einen ersten Harndrang ab einem Volumen von 150 bis 200 ml an, wobei die Werte je nach Alter, Geschlecht und Untersuchungsbedingungen variieren. Der erste Harndrang ist temporär, mit zunehmender Blasenfüllung wird ein stärkerer Harndrang angegeben, der das Erreichen der maximalen Blasenkapazität (zwischen 350 und 450 ml) signalisiert. Jede weitere Volumenzunahme hat eine deutliche, mitunter schmerzhafte Steigerung des Harndranggefühls zur Folge und geht mit einem vermehrten Druckanstieg aufgrund der ausgeschöpften Blasendehnungskapazität einher - die *Compliance* sinkt.

Unter Normalbefund bleibt im Allgemeinen während des gesamten Zeitraums der Blasenfüllung die Detrusoraktivität stabil und ändert sich im Allgemeinen auch nicht unter Provokation (Niesen, Husten, Stehen usw.). Es hat sich gezeigt, dass auch geringe Detrusorkontraktionen klinisch relevant sein können. Bis zum Erreichen der maximalen Blasenkapazität nimmt unter Normalbefund die Beckenbodenaktivität kontinuierlich zu, wobei zusätzlich intraabdominelle und intravesikale Druckerhöhungen, beispielsweise durch einen Hustenstoß, mit einer entsprechenden Aktivitätsvermehrung beantwortet werden. Bei einer vorliegend insbesondere neurogenen Blasenfunktionsstörung liegen demgegenüber vom Normalbefund abweichende Symptome bzw. Parameter vor, und zwar auch was die Detrusoraktivität und *Compliance* anbelangt.

Die Entleerungsphase kann durch Druck-Fluss-Studien analysiert werden. Die Miktion wird willkürlich oder unwillkürlich eingeleitet. Dabei kann sowohl die Detrusor- als auch die Urethralsphinkterfunktion beurteilt werden. Während der Entleerungsphase sind die wesentlichen urodynamischen Parameter Harnflussrate und Miktionsdruck.

Bei der Harnflussmessung werden in der nicht-invasiven Uroflowmetrie insbesondere die Flussrate in (ml/s), das Miktionsvolumen, die maximale Flussrate (Qₘₐₓ) und die Miktionszeit angegeben. Hinzu kommen Angaben zu den Miktionsdrücken selbst, wie z.B. Öffnungsdruck, Öffnungszeit bis zum Einsetzen des Harnflusses, Detrusordruck bei maximaler Flussrate (p_{det} bei Qₘₐₓ) und Verschlussdruck.

Anschließend kann eine Bestimmung des Restharns erfolgen. Dies ist möglich durch Subtraktion des Miktionsvolumens vom Füllungsvolumen oder durch Messung des Restharns durch Katheterisierung was in der Regel genauer ist, da die Eigendiurese mitberechnet wird.

Für weitere diesbezügliche Ausführungen insbesondere zur Bestimmung der erfindungsgemäß in Bezug genommenen urodynamischen Kenngrößen und dergleichen kann verwiesen werden auf Schultz-Lampel, Goepel, Haferkamp, "Urodynamik", dritte Auflage, 2012, Springer Verlag, insbesondere Seiten 111 bis 114, sowie auf die dort referierte Literatur, wobei der diesbezügliche Offenbarungsgehalt hiermit durch Bezugnahme vollumfänglich eingeschlossen ist.

Nachfolgend wird die vorliegende Erfindung weiterführend beschrieben:
Erfindungsgemäß verhält es sich insbesondere derart, dass die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck als Zielparameter unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, bzw. mit der Maßgabe und/oder Zielsetzung, dass der Detrusorkoeffizient (*Compliance*) als Zielparameter unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

Diesbezüglich kann beispielsweise zur Bestimmung der entsprechenden Parameter mit (Blasen-)Füllgeschwindigkeiten von 10 ml/min bis 30 ml/min, bevorzugt etwa 20 ml/min, vorgegangen werden.

Mit anderen Worten verhält es sich im Hinblick auf die obigen Ausführungen im Rahmen der vorliegenden Erfindung somit insbesondere derart, dass die Behandlungsdosis, insbesondere die tagesbezogene Therapiedosis, in ziel- bzw. zweckgerichteter Weise festgelegt wird, und zwar dahingehend, dass der Detrusordruck bzw. der Detrusorkoeffizient (*Compliance*) als jeweilige sozusagen pharmakologisch-medikamentös einzustellende Zielparameter auf einen konkreten Wert eingestellt werden. Infolge der gezielten Beeinflussung der in Rede stehenden Parameter wird neben der Verbesserung der Harnblasenfunktion insgesamt auch ein weiterführender Schutz des oberen Harntrakts bzw. der Nieren ermöglicht, wobei zudem die Kontinenz verbessert werden kann.

Gemäß einer erfindungsgemäßen Ausführungsform kann es sich zudem derart verhalten, dass die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck als Zielparameter unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und mit der Maßgabe und/oder Zielsetzung, dass der Detrusorkoeffizient *(Compliance)* als Zielparameter unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

Im Allgemeinen verhält es sich im Rahmen der vorliegenden Erfindung insbesondere derart, dass der Detrusordruck bestimmt bzw. berechnet wird als Differenz aus intravesikalem Druck (Harnblasendruck) einerseits und Rektaldruck (Abdominaldruck) andererseits. Dabei kann der intravesikale Druck (Harnblasendruck) insbesondere mittels urodynamischer Messung bestimmt werden, vorzugsweise während der insbesondere kontinuierlichen Harnblasenfüllung (Füllphase) der urodynamischen Messung. In diesem Zusammenhang kann zudem der Rektaldruck (Abdominaldruck) mittels (Druck-) Messung bestimmt werden, vorzugsweise während der insbesondere kontinuierlichen Harnblasenfüllung (Füllphase) der urodynamischen Messung.

Insbesondere gilt somit Detrusordruck = intravesikaler Druck (Harnblasendruck) - Rektaldruck.

Erfindungsgemäß verhält es sich zudem insbesondere derart, dass der Detrusorkoeffizient (*Compliance*) bestimmt und/oder berechnet wird als Quotient aus der Füllungsvolumenzunahme, insbesondere während der insbesondere kontinuierlichen Harnblasenfüllung (Füllphase), einerseits und dem korrelierenden intravesikalen Druckanstieg (Anstieg des Harnblasendrucks) anderseits. In diesem Zusammenhang kann der intravesikale Druck (Harnblasendruck) insbesondere mittels urodynamischer Messung bestimmt werden, vorzugsweise während der insbesondere kontinuierlichen Harnblasenfüllung (Füllphase) der urodynamischen Messung. Insbesondere gilt somit Detrusorkoeffizient (*Compliance)* = Füllungsvolumenzunahme (ΔV [ml]) / korrelierender intravesikaler Druckanstieg (Δ p [cm H₂O]).

Insbesondere wird im Rahmen der vorliegenden Erfindung der Detrusordruck als maximaler Detrusordruck, insbesondere maximaler Detrusordruck p_{Detrusor, max.} bzw. p_{Det, max.}, bestimmt bzw. herangezogen. Somit verhält es sich im Rahmen der erfindungsgemäßen Konzeption insbesondere derart, dass der maximale Detrusordruck P_{Detrusor, max.} unter Therapie mit der oxybutyninhaltigen Zusammensetzung auf einen Wert von höchstens 40 cm H₂O (40 Zentimeter Wassersäule) eingestellt wird.

Erfindungsgemäß verhält es sich zudem insbesondere derart, dass für den Fall, dass der Detrusordruck unter Therapie mit der oxybutyninhaltigen Zusammensetzung mehr als 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder für den Fall, dass der Detrusorkoeffizient (*Compliance*) unter Therapie mit der oxybutyninhaltigen Zusammensetzung weniger als 25 ml / cm H₂O (25 Milliliter pro Zentimeter Wassersäule), insbesondere weniger als 20 ml / cm H₂O (20 Milliliter pro Zentimeter Wassersäule), beträgt, die Behandlungsdosis, insbesondere die tagesbezogene Therapiedosis, bezogen auf das Oxybutynin, bevorzugt Oxybutyninhydrochlorid, angepasst, insbesondere erhöht wird, vorzugsweise mit den zuvor definierten Maßgaben und/oder Zielsetzungen (nämlich insbesondere dass der Detrusordruck unter Therapie dann höchstens 40 cm H₂O bzw. der Detrusorkoeffizient (*Compliance*) unter Therapie dann mindestens 20 ml / Zentimeter H₂O beträgt).

Erfindungsgemäß kann es sich gleichermaßen derart verhalten, dass für den Fall, dass der Detrusordruck unter Therapie mit der oxybutyninhaltigen Zusammensetzung mehr als 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und für den Fall, dass der Detrusorkoeffizient (*Compliance*) unter Therapie mit der oxybutyninhaltigen Zusammensetzung weniger als 25 ml / cm H₂O (25 Milliliter pro Zentimeter Wassersäule), insbesondere weniger als 20 ml / cm H₂O (20 Milliliter pro Zentimeter Wassersäule), beträgt, die Behandlungsdosis, insbesondere die tagesbezogene Therapiedosis, bezogen auf das Oxybutynin, bevorzugt Oxybutyninhydrochlorid, angepasst, insbesondere erhöht wird, vorzugsweise mit den zuvor definierten Maßgaben und/oder Zielsetzungen (nämlich insbesondere dass der Detrusordruck unter Therapie dann höchstens 40 cm H₂O bzw. der Detrusorkoeffizient (*Compliance*) unter Therapie dann mindestens 20 ml / Zentimeter H₂O beträgt).

Gemäß einer erfindungsgemäßen Ausführungsform verhält es sich in Bezug auf die zuvor angeführten Kenngrößen derart, dass (i) die urodynamische Kenngröße(n) (Messgröße(n)) ausgewählt ist bzw. sind aus der Gruppe von Detrusordruck und Detrusorkoeffizient (*Compliance*) sowie deren Kombination. Mit anderen Worten wird erfindungsgemäß in bevorzugter Weise auf den Detrusordruck und/oder den Detrusorkoeffizienten *(Compliance)* als diesbezügliche Kenngröße(n) (i) abgestellt.

Zudem kann (i) die urodynamische Kenngröße(n) (Messgröße(n)) mindestens eine weitere urodynamische Kenngröße (Messgröße) (i) umfassen, wobei die weitere urodynamische Kenngröße ausgewählt sein kann aus der Gruppe von maximaler Harnblasenkapazität, insbesondere zystometrischer maximaler Harnblasenkapazität, Harnblasenfüllungssensitivität und Gefühl des ersten Harndrangs sowie deren Kombinationen.

Insbesondere kann es sich erfindungsgemäß derart verhalten, dass (i) die urodynamische Kenngrößen (Messgrößen) ausgewählt sind aus der Gruppe von Detrusordruck und Detrusorkoeffizient (*Compliance*) sowie deren Kombination und zudem mindestens eine weitere urodynamische Kenngröße (Messgröße) (i) umfassen, wobei die weitere urodynamische Kenngröße ausgewählt ist aus der Gruppe von maximaler Harnblasenkapazität, insbesondere zystometrischer maximaler Harnblasenkapazität, Harnblasenfüllungssensitivität und Gefühl des ersten Harndrangs sowie deren Kombinationen.

In diesem Zusammenhang kann es erfindungsgemäß weiterhin auch vorgesehen sein, dass die Behandlungsdosis, insbesondere die tagesbezogene Therapiedosis, bezogen auf das Oxybutynin, bevorzugt Oxybutyninhydrochlorid, in Abhängigkeit von mindestens einer weiteren urodynamischen Kenngröße (Messgröße) (i) des zu behandelnden Patienten bestimmt und/oder festgelegt wird, wobei die weitere urodynamische Kenngröße ausgewählt ist aus der Gruppe von maximaler Harnblasenkapazität, insbesondere zystometrischer maximaler Harnblasenkapazität, Harnblasenfüllungssensitivität und Gefühl des ersten Harndrangs sowie deren Kombinationen.

Die vorgenannten weiteren Kenngrößen bzw. Parameter erlauben in gezielter Kombination mit den objektiven urodynamischen Kenngrößen in Form des Detrusordrucks, Detrusorkoeffizienten bzw. des intravesikalen Drucks eine weiterführende Spezifizierung der zugrundeliegenden Behandlungsdosis, und zwar insbesondere insofern, als die Kombination der zuvor genannten Kenngrößen bzw. Parameter eine valide Gesamtaussage ermöglicht, und dies sogar vor dem Hintergrund, dass es sich bei den weiteren Kenngrößen in Form der maximalen Harnblasenkapazität, der Harnblasenfüllungsaktivität sowie des Gefühls des ersten Harndrangs insbesondere um subjektive Kenngrößen bzw. Parameter handelt.

Darüber hinaus können im Rahmen der vorliegenden Erfindung die jeweiligen Kenngrößen (i), (ii), (iii), (iv) bzw. (v) im Hinblick auf die Bestimmung bzw. Festlegung der Behandlungsdosis wie folgt herangezogen werden:
So kann es im Rahmen der vorliegenden Erfindung vorgesehen sein, dass die tagesbezogene Therapiedosis, bezogen auf das Oxybutynin, bevorzugt Oxybutyninhydrochlorid, in Abhängigkeit der nachfolgend angeführten klinischen, medizinischen und/oder anamnestischen patientenindividuellen Kenngröße (i) bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen; insbesondere ausgewählt aus der Gruppe von Detrusordruck und Detrusorkoeffizient (*Compliance*) sowie deren Kombination.

Somit kann erfindungsgemäß auf die Kenngröße(n) (i) alleinig bzw. als solche zur Bestimmung bzw. Festlegung der Behandlungsdosis abgestellt werden.

Weiterhin kann es sich erfindungsgemäß aber auch derart verhalten, dass die Behandlungsdosis, insbesondere die tagesbezogene Therapiedosis, bezogen auf das Oxybutynin, bevorzugt Oxybutyninhydrochlorid, in Abhängigkeit von einer Kombination mindestens der nachfolgend angeführten klinischen, medizinischen und/oder anamnestischen patientenindividuellen Kenngrößen (i) und (ii) des zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen; insbesondere ausgewählt aus der Gruppe von Detrusordruck und Detrusorkoeffizient (*Compliance*) sowie deren Kombination; und
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten.

Somit kann diesbezüglich auf eine Kombination der Kenngröße(n) (i) und (ii) abgestellt werden.

Erfindungsgemäß ist es zudem möglich, dass die Behandlungsdosis, insbesondere die tagesbezogene Therapiedosis, bezogen auf das Oxybutynin, bevorzugt Oxybutyninhydrochlorid, in Abhängigkeit von einer Kombination mindestens der nachfolgend angeführten klinischen, medizinischen und/oder anamnestischen patientenindividuellen Kenngrößen (i) bis (iii) des zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen; insbesondere ausgewählt aus der Gruppe von Detrusordruck und Detrusorkoeffizient (*Compliance*) sowie deren Kombination;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten; und
(iii) Alter des zu behandelnden Patienten.

Diesbezüglich kann somit auf eine Kombination der Kenngrößen (i), (ii) und (iii) abgestellt werden.

Weiterhin kann erfindungsgemäß auch eine Kombination der Kenngrößen (i) bis (iv) zur Bestimmung bzw. Festlegung der Behandlungsdosis herangezogen werden. Somit kann es erfindungsgemäß vorgesehen sein, dass die Behandlungsdosis, insbesondere die tagesbezogene Therapiedosis, bezogen auf das Oxybutynin, bevorzugt Oxybutyninhydrochlorid, in Abhängigkeit von einer Kombination mindestens der nachfolgend angeführten klinischen, medizinischen und/oder anamnestischen patientenindividuellen Kenngrößen (i) bis (iv) des zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient *(Compliance)* und intravesikalem Druck (Harnblasendruck) sowie deren Kombinationen; insbesondere ausgewählt aus der Gruppe von Detrusordruck und Detrusorkoeffizient *(Compliance)* sowie deren Kombination;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten; und
(iv) Art und/oder Schwere der pathologischen Blasenanatomie.

Somit kann auch auf eine Kombination der Kenngrößen (i), (ii), (iii) und (iv) abgestellt werden.

Schließlich können auch die Kenngrößen (i), (ii), (iii), (iv) und (v) in ihrer Gesamtheit zur Bestimmung bzw. Festlegung der Behandlungsdosis herangezogen werden. Somit kann es erfindungsgemäß vorgesehen sein, dass die Behandlungsdosis, insbesondere die tagesbezogene Therapiedosis, bezogen auf das Oxybutynin, bevorzugt Oxybutyninhydrochlorid, in Abhängigkeit von einer Kombination mindestens der nachfolgend angeführten klinischen, medizinischen und/oder anamnestischen patientenindividuellen Kenngrößen (i) bis (v) des zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen; insbesondere ausgewählt aus der Gruppe von Detrusordruck und Detrusorkoeffizient (*Compliance*) sowie deren Kombination;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und
(v) Status der Nierenfunktion.

Darüber hinaus kann es im Rahmen der vorliegenden Erfindung auch vorgesehen sein, dass die insbesondere neurogenen Blasenfunktionsstörungen mit einer spastischen Harnblase einhergehen bzw. hierdurch gekennzeichnet sind. Eine spastisch ausgebildete Harnblase kann dabei insbesondere auch mit einer Detrusorhyperreflexie einhergehen. Zudem liegt bei einer spastischen Harnblase oftmals eine verringerte Blasenkapazität vor. Diesbezüglich liegt auch oftmals eine Detrusorüberaktivität vor, wobei zudem Blasenkontraktionen und Relaxation des äußeren Schließmuskels typischerweise nicht koordiniert sind, so dass auch diesbezüglich eine Detrusor-Sphinkter-Dyssynergie vorliegen kann.

Erfindungsgemäß kann es sich zudem derart verhalten, dass die insbesondere neurogenen Blasenfunktionsstörungen mit einer hypersensitiven, hypokapazitären, instabilen und/oder hypertonen Harnblase einhergehen bzw. hierdurch gekennzeichnet sind.

Was die der vorliegenden Erfindung zugrundeliegenden und zu behandelnden insbesondere neurogenen Blasenfunktionsstörungen weiterhin anbelangt, so gehen diese insbesondere mit einem erhöhten Detrusordruck einher bzw. sind hierdurch gekennzeichnet. In diesem Zusammenhang kann es insbesondere der Fall sein, dass der Detrusordruck im unbehandelten Zustand und/oder ohne Therapie mit der oxybutyninhaltigen Zusammensetzung mehr als 40 cm H₂O (40 Zentimeter Wassersäule) beträgt. In diesem Zusammenhang wird der Detrusordruck insbesondere bestimmt mittels urodynamischer Messung, vorzugsweise während der insbesondere kontinuierlichen Harnblasenfüllung (Füllphase) der urodynamischen Messung, wie zuvor angeführt.

Zudem ist es insbesondere der Fall, dass die neurogenen Blasenfunktionsstörungen als solche mit einem verringertem Detrusorkoeffizienten (*Compliance*) bzw. mit einer *Low-Compliance*-Harnblase einhergehen bzw. hierdurch gekennzeichnet sind. Diesbezüglich kann es insbesondere der Fall sein, dass der Detrusorkoeffizient (*Compliance*) im unbehandelten Zustand und/oder ohne Therapie mit der oxybutyninhaltigen Zusammensetzung weniger als 25 ml / cm H₂O (25 Milliliter pro Zentimeter Wassersäule), insbesondere weniger als 20 ml / cm H₂O (20 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung, wie zuvor angeführt.

Im Lichte der obigen Ausführungen verhält es sich im Rahmen der erfindungsgemäßen Konzeption mit der Dosisfindung bzw. Dosisoptimierung insbesondere derart, dass sowohl der Detrusordruck als auch der Detrusorkoeffizient optimal eingestellt werden können, was zu einer signifikanten Besserung des Krankheitsbildes der neurogenen Blasenfunktionsstörungen, wie vorliegend definiert, führt.

Darüber hinaus kann die erfindungsgemäße Zusammensetzung die nachfolgend angeführten Eigenschaften aufweisen:
- So kann die Zusammensetzung das Oxybutynin, bevorzugt das Oxybutyninhydrochlorid (Oxybutynin-HCl), in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, enthalten. Erfindungsgemäß kann es gleichermaßen vorgesehen sein, dass die Zusammensetzung das Oxybutynin, bevorzugt das Oxybutyninhydrochlorid (Oxybutynin-HCl) in einer Konzentration von 1 mg / ml enthält.
- Zudem kann die Zusammensetzung das Elektrolytsalz, bevorzugt Natriumchlorid, berechnet als Elektrolytkation, bevorzugt berechnet als Natrium im Fall von Natriumchlorid, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml, enthalten. In diesem Zusammenhang kann die Zusammensetzung das Elektrolytsalz, bevorzugt Natriumchlorid, berechnet als Elektrolytkation, bevorzugt berechnet als Natrium im Fall von Natriumchlorid, in einer Konzentration von 3,5 mg / ml enthalten.
- Zudem kann die Zusammensetzung einen pH-Wert im Bereich von 3 bis 5, insbesondere im Bereich von 3,8 bis 4,5, aufweisen. In diesem Zusammenhang kann der pH-Wert der Zusammensetzung unter Verwendung von bzw. mittels Salzsäure eingestellt sein.

Darüber hinaus kann es im Rahmen der vorliegenden Erfindung vorgesehen sein, dass die Zusammensetzung zur Verabreichung, insbesondere Instillation in die Harnblase, eines Volumens der Zusammensetzung im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, vorliegt. Diesbezüglich kann die Zusammensetzung mit einem Volumen der Zusammensetzung im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, verabreicht werden. In diesem Zusammenhang können auch entsprechende Wirkmengen bzw. Wirkstoffdosen verabreicht werden.

Im Nachfolgenden werden weitere Ausführungen zu der Dosierung der der Zusammensetzung nach der Erfindung zugrundeliegenden Wirksubstanz in Form von Oxybutynin bzw. Oxybutyninhydrochlorid angegeben. Diesbezüglich kann es grundsätzlich vorgesehen sein, von den nachfolgend genannten Werten abzuweichen, ohne dass der Rahmen der vorliegenden Erfindung verlassen ist. Dies liegt im Ermessen des Fachmanns in jedem Einzelfall. Hinsichtlich der auf den Tag bezogenen Dosierung wird nachfolgend die Abkürzung bzw. Maßeinheit "Dosis (z.B. mg)/d" (mit "d" für *diem = Tag* bzw. für 24 h) verwendet. Die nachfolgend genannten Dosen sind dabei vor dem Hintergrund der vorgenannten Maßgaben bzw. Zielsetzungen zu variieren bzw. anzupassen.

Im Allgemeinen kann im Rahmen der vorliegenden Erfindung die Behandlungsdosis, insbesondere die tagesbezogene Therapiedosis, bezogen auf das Oxybutynin, bevorzugt Oxybutyninhydrochlorid (Oxybutynin-HCl), und bezogen auf das Körpergewicht ([kg]), im Bereich von 0,05 mg/(kg·d) bis 1,5 mg/(kg·d), insbesondere im Bereich von 0,1 mg/(kg·d) bis 1,25 mg/(kg·d), vorzugsweise im Bereich von 0,125 mg/(kg·d) bis 1 mg/(kg·d), bevorzugt im Bereich von 0,14 mg/(kg·d) bis 0,9 mg/(kg·d), liegen.

Insbesondere kann die Zusammensetzung in diesem Zusammenhang zur Verabreichung, insbesondere Instillation in die Harnblase, der Behandlungsdosis, insbesondere der tagesbezogenen Therapiedosis, bezogen auf das Oxybutynin, bevorzugt Oxybutyninhydrochlorid (Oxybutynin-HCl), und bezogen auf das Körpergewicht ([kg]), im Bereich von 0,05 mg/(kg·d) bis 1,5 mg/(kg·d), insbesondere im Bereich von 0,1 mg/(kg·d) bis 1,25 mg/(kg·d), vorzugsweise im Bereich von 0,125 mg/(kg·d) bis 1 mg/(kg·d), bevorzugt im Bereich von 0,14 mg/(kg·d) bis 0,9 mg/(kg·d), hergerichtet sein. Diesbezüglich kann die Zusammensetzung mit der Behandlungsdosis, insbesondere der tagesbezogenen Therapiedosis, bezogen auf das Oxybutynin, bevorzugt Oxybutyninhydrochlorid (Oxybutynin-HCl), und bezogen auf das Körpergewicht ([kg]) im Bereich von 0,05 mg/(kg·d) bis 1,5 mg/(kg·d), insbesondere im Bereich von 0,1 mg/(kg·d) bis 1,25 mg/(kg·d), vorzugsweise im Bereich von 0,125 mg/(kg·d) bis 1 mg/(kg·d), bevorzugt im Bereich von 0,14 mg/(kg·d) bis 0,9 mg/(kg·d), verabreicht werden.

Erfindungsgemäß kann es im Allgemeinen vorgesehen sein, dass die Behandlungsdosis, insbesondere die tagesbezogene Therapiedosis, bezogen auf das Oxybutynin, bevorzugt Oxybutyninhydrochlorid (Oxybutynin-HCl) und bezogen auf das Körpergewicht ([kg]), auf einen Bereich von 0,05 mg/(kg·d) bis 1,5 mg/(kg·d), insbesondere auf einen Bereich von 0,1 mg/(kg·d) bis 1,25 mg/(kg·d), vorzugsweise auf einen Bereich von 0,125 mg/(kg·d) bis 1 mg/(kg·d), bevorzugt auf einen Bereich von 0,14 mg/(kg·d) bis 0,9 mg/(kg·d), bestimmt und/oder festgelegt wird.

Weiterhin kann es erfindungsgemäß im Allgemeinen vorgesehen sein, dass die (körpergewichtsunabhängige bzw. absolute) Behandlungsdosis, insbesondere die tagesbezogene Therapiedosis, bezogen auf das Oxybutynin, bevorzugt Oxybutyninhydrochlorid (Oxybutynin-HCl), im Bereich von 1 mg/d bis 100 mg/d, insbesondere im Bereich von 2 mg/d bis 90 mg/d, vorzugsweise im Bereich von 3 mg/d bis 75 mg/d, bevorzugt im Bereich von 4 mg/d bis 65 mg/d, besonders bevorzugt im Bereich von 5 mg/d bis 60 mg/d, liegt.

Gleichermaßen kann es im Allgemeinen vorgesehen sein, dass die Zusammensetzung zur Verabreichung, insbesondere Instillation in die Harnblase, der (körpergewichtsunabhängige bzw. absolute) Behandlungsdosis, insbesondere der tagesbezogenen Therapiedosis, bezogen auf das Oxybutynin, bevorzugt Oxybutyninhydrochlorid (Oxybutynin-HCl), im Bereich von 1 mg/d bis 100 mg/d, insbesondere im Bereich von 2 mg/d bis 90 mg/d, vorzugsweise im Bereich von 3 mg/d bis 75 mg/d, bevorzugt im Bereich von 4 mg/d bis 65 mg/d, besonders bevorzugt im Bereich von 5 mg/d bis 60 mg/d, hergerichtet ist. Diesbezüglich kann die Zusammensetzung mit der Behandlungsdosis, insbesondere der tagesbezogenen Therapiedosis, bezogen auf das Oxybutynin, bevorzugt Oxybutyninhydrochlorid (Oxybutynin-HCl), im Bereich von 1 mg/d bis 100 mg/d, insbesondere im Bereich von 2 mg/d bis 90 mg/d, vorzugsweise im Bereich von 3 mg/d bis 75 mg/d, bevorzugt im Bereich von 4 mg/d bis 65 mg/d, besonders bevorzugt im Bereich von 5 mg/d bis 60 mg/d, verabreicht werden.

Erfindungsgemäß kann es sich somit insgesamt derart verhalten, dass die (körpergewichtsunabhängige bzw. absolute) Behandlungsdosis, insbesondere die tagesbezogene Therapiedosis, bezogen auf das Oxybutynin, bevorzugt Oxybutyninhydrochlorid (Oxybutynin-HCl), auf einen Bereich von 1 mg/d bis 100 mg/d, insbesondere im Bereich von 2 mg/d bis 90 mg/d, vorzugsweise im Bereich von 3 mg/d bis 75 mg/d, bevorzugt im Bereich von 4 mg/d bis 65 mg/d, besonders bevorzugt im Bereich von 5 mg/d bis 60 mg/d, bestimmt bzw. festgelegt wird.

Weiterhin kann es im Rahmen der vorliegenden Erfindung vorgesehen sein, dass die Behandlungsdosis, insbesondere die tagesbezogene Therapiedosis, bezogen auf das Oxybutynin, bevorzugt Oxybutyninhydrochlorid (Oxybutynin-HCl), in Abhängigkeit vom (Patienten-)Alter und/oder altersgruppenabhängig in den folgenden Bereichen liegt bzw.
dass die Zusammensetzung zur Verabreichung, insbesondere Instillation in die Harnblase, der Behandlungsdosis, insbesondere der tagesbezogenen Therapiedosis, bezogen auf das Oxybutynin, bevorzugt Oxybutyninhydrochlorid (Oxybutynin-HCl), in den folgenden Bereichen hergerichtet ist bzw.
dass die Zusammensetzung mit der Behandlungsdosis, insbesondere der tagesbezogenen Therapiedosis, bezogen auf das Oxybutynin, bevorzugt Oxybutyninhydrochlorid (Oxybutynin-HCl), in den folgenden Bereichen verabreicht wird bzw.
dass die Behandlungsdosis, insbesondere die tagesbezogene Therapiedosis, bezogen auf das Oxybutynin, bevorzugt Oxybutyninhydrochlorid (Oxybutynin-HCl), auf die folgenden Bereiche bestimmt und/oder festgelegt bzw.:
- Altersgruppe von 6 bis 12 Jahren (Kinder):
   1 mg/d bis 50 mg/d, insbesondere 2 mg/d bis 50 mg/d, vorzugsweise 2,5 mg/d bis 45 mg/d, bevorzugt 5 mg/d bis 40 mg/d, besonders bevorzugt 5 mg/d bis 30 mg/d;
- Altersgruppe von 12 bis 18 Jahren (Jugendliche/Heranwachsende):
   5 mg/d bis 80 mg/d, insbesondere 7,5 mg/d bis 70 mg/d, vorzugsweise 10 mg/d bis 60 mg/d;
- Altersgruppe von 19 bis 65 Jahren (Erwachsene):
   5 mg/d bis 80 mg/d, insbesondere 7,5 mg/d bis 70 mg/d, vorzugsweise 10 mg/d bis 60 mg/d;
- Altersgruppe ab 65 Jahre (ältere Personen):
   5 mg/d bis 45 mg/d, insbesondere 7,5 mg/d bis 40 mg/d, vorzugsweise 10 mg/d bis 30 mg/d.

Somit kann eine altersbezogene Dosierung erfolgen, und zwar auch im Hinblick auf die zugrundeliegenden Kenngrößen (i) bis (v), wie zuvor definiert bzw. unter Einbezug der zuvor definierten Maßgaben bzw. Zielsetzungen.

Erfindungsgemäß kann es zudem vorgesehen sein, dass die Zusammensetzung zur Verabreichung der Behandlungsdosis, insbesondere der tagesbezogenen Therapiedosis, in Form einer täglichen Einmalgabe (Tageseinmalgabe) und/oder Einzelgabe oder aber über den Tag verteilt und/oder in Form mehrfacher über den Tag verteilter Teilgaben (mehrtäglicher Teilgaben), insbesondere in Form von 2 bis 10 Teilgaben, insbesondere 2 bis 8 Teilgaben, vorzugsweise 2 bis 6 Teilgaben, hergerichtet ist. Dabei kann die Behandlungsdosis, insbesondere die tagesbezogene Therapiedosis, in Form einer täglichen Einmalgabe und/oder Einzelgabe oder aber über den Tag verteilt und/oder in Form mehrtäglicher Teilgaben, insbesondere in Form von 2 bis 10 Teilgaben, insbesondere 2 bis 8 Teilgaben, vorzugsweise 2 bis 6 Teilgaben, verabreicht werden.

Die diesbezügliche Dosierung bzw. die Verabreichung der Behandlungsdosis, insbesondere der tagesbezogenen Therapiedosis, kann somit mittels einer täglichen Einmalgabe oder aber über den Tag verteilt und somit auf Basis entsprechender über den Tag verteilter Einzelbehandlungsdosen erfolgen. Dabei kann eine jeweilige Einmalgabe bzw. einzelne Teilgabe beispielsweise eine Dosis bzw. Wirkstoffmenge, bezogen auf das Oxybutynin, bevorzugt Oxybutyninhydrochlorid, von 5 mg oder aber von 10 mg aufweisen.

Insbesondere im Hinblick auf die Bestimmung bzw. Festlegung der Behandlungsdosis kann im Rahmen der vorliegenden Erfindung auch wie folgt vorgegangen werden.

So kann es erfindungsgemäß vorgesehen sein, dass zunächst und/oder zu Beginn der Behandlung und/oder zu Zwecken der Auffindung, insbesondere Bestimmung und/oder Festlegung, der Behandlungsdosis, insbesondere der tagesbezogenen Therapiedosis, eine Initialbehandlungsdosis (Anfangsdosis), insbesondere tagesbezogene Initialtherapiedosis (tagesbezogene Anfangstherapiedosis), bezogen auf das Oxybutynin, bevorzugt Oxybutyninhydrochlorid (Oxybutynin-HCl), insbesondere täglich und für einen definierten Initialbehandlungszeitraum, verabreicht wird und insbesondere innerhalb des Initialbehandlungszeitraums in Abhängigkeit von den Kenngrößen (i), (ii), (iii), (iv) und/oder (v), insbesondere in Abhängigkeit zumindest von der Kenngröße (i), angepasst wird, insbesondere erhöht wird, insbesondere so dass die Behandlungsdosis, insbesondere die tagesbezogene Therapiedosis, hierdurch festgelegt und/oder bestimmt wird, vorzugsweise mit den zuvor definierten Maßgaben und/oder Zielsetzungen, und/oder insbesondere so dass hierdurch mindestens ein Zielparameter, wie zuvor definiert, erfüllt ist.

Mit anderen Worten kann erfindungsgemäß beispielsweise derart vorgegangen werden, dass die Initialbehandlungsdosis so lange angepasst bzw. erhöht wird, bis der Detrusordruck unter Therapie mit der oxybutyninhaltigen Zusammensetzung höchstens 40 Zentimeter H₂O (40 Zentimeter Wassersäule) beträgt, wie zuvor angeführt, und/oder bis der Detrusorkoeffizient mindestens 20 ml / cm H₂O (20 Milliliter pro Zentimeter Wassersäule) beträgt, wie gleichermaßen zuvor angeführt. Auf dieser Basis kann dann die Behandlungsdosis, insbesondere die tagesbezogene Therapiedosis, entsprechend übernommen bzw. festgelegt werden.

Die Initialdosis kann gleichermaßen als Übergangsdosis verstanden werden, welche sozusagen zur Behandlungsdosis führt, wobei die Behandlungsdosis insbesondere dem Wert der Initialbehandlungsdosis bzw. Übergangsdosis entsprechen kann, welcher zu der zuvor angeführten Einstellung der Kenngrößen, und zwar insbesondere des Detrusordrucks bzw. des Detrusorkoeffizienten, wie zuvor definiert, führt.

Im Allgemeinen kann die Initialbehandlungsdosis bzw. die Übergangsbehandlungsdosis, insbesondere die tagesbezogene Initialtherapiedosis bzw. Übergangstherapiedosis, über einen Zeitraum beispielsweise von 0,5 Wochen bis 10 Wochen verabreicht werden, insbesondere gefolgt von der Verabreichung der Behandlungsdosis, insbesondere der tagesbezogenen Therapiedosis (welche gleichermaßen im weiteren zeitlichen Verlauf im Lichte der vorliegenden Maßgaben bzw. Zielsetzungen weiterführend bestimmt bzw. festgelegt bzw. angepasst werden kann).

Im Rahmen der vorliegenden Erfindung kann es dabei insbesondere vorgesehen sein, dass die Initialbehandlungsdosis, insbesondere die tagesbezogene Initialtherapiedosis, kleiner ist als die Behandlungsdosis, insbesondere die tagesbezogenen Therapiedosis.

Diesbezüglich kann die Initialbehandlungsdosis, insbesondere die tagesbezogene Initialtherapiedosis, höchstens 100 %, insbesondere höchstens 80 %, vorzugsweise höchstens 60 %, bevorzugt höchstens 40 %, besonders bevorzugt höchstens 20 %, der Behandlungsdosis, insbesondere der tagesbezogenen Therapiedosis, betragen.

Darüber hinaus kann die Initialbehandlungsdosis, insbesondere die tagesbezogene Initialtherapiedosis, im Bereich von 1 % bis 100 %, insbesondere im Bereich von 2 % bis 80 %, vorzugsweise im Bereich von 3 % bis 60 %, bevorzugt im Bereich von 4 % bis 40 %, besonders bevorzugt im Bereich von 5 % bis 20 %, der Behandlungsdosis, insbesondere der tagesbezogenen Therapiedosis, liegen.

Was die Initialbehandlungsdosis bzw. die tagesbezogene Initialtherapiedosis weiterhin anbelangt, so kann diese insbesondere auf das Oxybutynin, bevorzugt Oxybutyninhydrochlorid (Oxybutynin-HCl), und bezogen auf das Körpergewicht ([kg]), im Bereich von 0,025 mg/(kg·d) bis 1,25 mg/(kg·d), insbesondere im Bereich von 0,03 mg/(kg·d) bis 1 mg/(kg·d), vorzugsweise im Bereich von 0,04 mg/(kg·d) bis 0,8 mg/(kg·d), bevorzugt im Bereich von 0,05 mg/(kg·d) bis 0,6 mg/(kg·d), liegen.

Diesbezüglich kann es erfindungsgemäß somit vorgesehen sein, dass die Zusammensetzung zur Verabreichung, insbesondere Instillation in die Harnblase, der Initialbehandlungsdosis, insbesondere der tagesbezogenen Initialtherapiedosis, bezogen auf das Oxybutynin, bevorzugt Oxybutyninhydrochlorid (Oxybutynin-HCl), und bezogen auf das Körpergewicht ([kg]), im Bereich von 0,025 mg/(kg·d) bis 1,25 mg/(kg·d), insbesondere im Bereich von 0,03 mg/(kg·d) bis 1 mg/(kg·d), vorzugsweise im Bereich von 0,04 mg/(kg·d) bis 0,8 mg/(kg·d), bevorzugt im Bereich von 0,05 mg/(kg·d) bis 0,6 mg/(kg·d), hergerichtet ist bzw. dass die Zusammensetzung mit der Initialbehandlungsdosis, insbesondere der tagesbezogenen Initialtherapiedosis, bezogen auf das Oxybutynin, bevorzugt Oxybutyninhydrochlorid (Oxybutynin-HCl), und bezogen auf das Körpergewicht ([kg]), im Bereich im Bereich von 0,025 mg/(kg·d) bis 1,25 mg/(kg·d), insbesondere im Bereich von 0,03 mg/(kg·d) bis 1 mg/(kg·d), vorzugsweise im Bereich von 0,04 mg/(kg·d) bis 0,8 mg/(kg·d), bevorzugt im Bereich von 0,05 mg/(kg·d) bis 0,6 mg/(kg·d), verabreicht wird.

Somit kann es erfindungsgemäß insgesamt vorgesehen sein, dass die Initialbehandlungsdosis, insbesondere die tagesbezogene Initialtherapiedosis, bezogen auf das Oxybutynin, bevorzugt Oxybutyninhydrochlorid (Oxybutynin-HCl), und bezogen auf das Körpergewicht ([kg]), auf einen Bereich von 0,025 mg/(kg·d) bis 1,25 mg/(kg·d), insbesondere auf einen Bereich von 0,03 mg/(kg·d) bis 1 mg/(kg·d), vorzugsweise auf einen Bereich von 0,04 mg/(kg·d) bis 0,8 mg/(kg·d), bevorzugt auf einen Bereich von 0,05 mg/(kg·d) bis 0,6 mg/(kg·d), bestimmt bzw. festgelegt wird.

Auch diesbezüglich erfolgt die Bestimmung bzw. Festlegung der Initialbehandlungsdosis insbesondere im Hinblick auf die zuvor angeführten Kenngrößen (i) bis (v), und zwar insbesondere was eine gezielte Senkung des Detrusordrucks bzw. eine gezielte Steigerung des Detrusorkoeffizienten unter Berücksichtigung der vorgenannten Maßgaben bzw. Zielsetzungen anbelangt.

Vor diesem Hintergrund kann es erfindungsgemäß gleichermaßen möglich sein, dass die (körpergewichtsunabhängige bzw. absolute) Initialbehandlungsdosis, insbesondere die tagesbezogene Initialtherapiedosis, bezogen auf das Oxybutynin, bevorzugt Oxybutyninhydrochlorid (Oxybutynin-HCl), im Bereich von 0,1 mg/d bis 80 mg/d, insbesondere im Bereich von 0,2 mg/d bis 75 mg/d, vorzugsweise im Bereich von 0,5 mg/d bis 50 mg/d, bevorzugt im Bereich von 0,75 mg/d bis 40 mg/d, besonders bevorzugt im Bereich von 1 mg/d bis 30 mg/d, liegt.

Diesbezüglich kann die Zusammensetzung zur Verabreichung, insbesondere Instillation in die Harnblase, der (körpergewichtsunabhängigen bzw. absoluten) Initialbehandlungsdosis, insbesondere der tagesbezogenen Initialtherapiedosis, bezogen auf das Oxybutynin, bevorzugt Oxybutyninhydrochlorid (Oxybutynin-HCl), im Bereich von 0,1 mg/d bis 80 mg/d, insbesondere im Bereich von 0,2 mg/d bis 75 mg/d, vorzugsweise im Bereich von 0,5 mg/d bis 50 mg/d, bevorzugt im Bereich von 0,75 mg/d bis 40 mg/d, besonders bevorzugt im Bereich von 1 mg/d bis 30 mg/d, hergerichtet sein. In diesem Zusammenhang kann die Zusammensetzung mit der Initialbehandlungsdosis, insbesondere der tagesbezogenen Initialtherapiedosis, bezogen auf das Oxybutynin, bevorzugt Oxybutyninhydrochlorid (Oxybutynin-HCl), im Bereich von 0,1 mg/d bis 80 mg/d, insbesondere im Bereich von 0,2 mg/d bis 75 mg/d, vorzugsweise im Bereich von 0,5 mg/d bis 50 mg/d, bevorzugt im Bereich von 0,75 mg/d bis 40 mg/d, besonders bevorzugt im Bereich von 1 mg/d bis 30 mg/d, verabreicht werden.

Somit kann die (körpergewichtsunabhängige bzw. absolute) Initialbehandlungsdosis, insbesondere die tagesbezogene Initialtherapiedosis, bezogen auf das Oxybutynin, bevorzugt Oxybutyninhydrochlorid (Oxybutynin-HCl), auf einen Bereich von 0,1 mg/d bis 80 mg/d, insbesondere auf einen Bereich von 0,2 mg/d bis 75 mg/d, vorzugsweise auf einen Bereich von 0,5 mg/d bis 50 mg/d, bevorzugt auf einen Bereich von 0,75 mg/d bis 40 mg/d, besonders bevorzugt auf einen Bereich von 1 mg/d bis 30 mg/d, bestimmt bzw. festgelegt werden.

Erfindungsgemäß kann es insbesondere vorgesehen sein, dass die Initialbehandlungsdosis, insbesondere die tagesbezogene Initialtherapiedosis, bezogen auf das Oxybutynin, bevorzugt Oxybutyninhydrochlorid (Oxybutynin-HCl), in Abhängigkeit vom (Patienten-)Alter und/oder altersgruppenabhängig in den folgenden Bereichen liegt bzw.
dass die Zusammensetzung zur Verabreichung, insbesondere Instillation in die Harnblase, der Initialbehandlungsdosis, insbesondere der tagesbezogenen Initialtherapiedosis, bezogen auf das Oxybutynin, bevorzugt Oxybutyninhydrochlorid (Oxybutynin-HCl), in den folgenden Bereichen hergerichtet ist bzw.
dass die Zusammensetzung mit der Initialbehandlungsdosis, insbesondere der tagesbezogenen Initialtherapiedosis, bezogen auf das Oxybutynin, bevorzugt Oxybutyninhydrochlorid (Oxybutynin-HCl), in den folgenden Bereichen verabreicht wird bzw.
dass die Initialbehandlungsdosis, insbesondere die tagesbezogene Initialtherapiedosis, bezogen auf das Oxybutynin, bevorzugt Oxybutyninhydrochlorid (Oxybutynin-HCl), auf die folgenden Bereiche bestimmt bzw. festgelegt wird:
- Altersgruppe von 6 bis 12 Jahren (Kinder):
   0,1 mg/d bis 40 mg/d, insbesondere 0,5 mg/d bis 30 mg/d, vorzugsweise 1 mg/d bis 20 mg/d;
- Altersgruppe von 12 bis 18 Jahren (Jugendliche/Heranwachsende):
   1 mg/d bis 60 mg/d, insbesondere 2 mg/d bis 50 mg/d, vorzugsweise 5 mg/d bis 40 mg/d;
- Altersgruppe von 19 bis 65 Jahren (Erwachsene):
   1 mg/d bis 60 mg/d, insbesondere 2 mg/d bis 50 mg/d, vorzugsweise 5 mg/d bis 40 mg/d;
- Altersgruppe ab 65 Jahre (ältere Personen):
   1 mg/d bis 35 mg/d, insbesondere 2 mg/d bis 20 mg/d, vorzugsweise 3 mg/d bis 10 mg/d.

Auch in Bezug auf die Initialbehandlungsdosis kann es vorgesehen sein, dass diese in Form einer täglichen Einmalgabe oder aber über den Tag verteilt in Form mehrfacher Teilgaben verabreicht wird.

So kann es erfindungsgemäß vorgesehen sein, dass die Zusammensetzung zur Verabreichung der Initialbehandlungsdosis, insbesondere der tagesbezogenen Initialtherapiedosis, in Form einer täglichen Einmalgabe (Tageseinmalgabe) und/oder Einzelgabe oder aber über den Tag verteilt und/oder in Form mehrfacher über den Tag verteilter Teilgaben (mehrtäglicher Teilgaben), insbesondere in Form von 2 bis 10 Teilgaben, insbesondere 2 bis 8 Teilgaben, vorzugsweise 2 bis 6 Teilgaben, hergerichtet ist. Insbesondere kann die Initialbehandlungsdosis, insbesondere die tagesbezogene Initialtherapiedosis, in Form einer täglichen Einmalgabe und/oder Einzelgabe oder aber über den Tag verteilt und/oder in Form mehrtäglicher Teilgaben, insbesondere in Form von 2 bis 10 Teilgaben, insbesondere 2 bis 8 Teilgaben, vorzugsweise 2 bis 6 Teilgaben, verabreicht werden. Dabei können die entsprechenden Einmalgaben bzw. Teilgaben entsprechende Dosen bzw. Wirkstoffmengen an Oxybutynin bzw. Oxybutyninhydrochlorid aufweisen, beispielsweise 5 mg oder 10 mg.

Erfindungsgemäß kann es zudem vorgesehen sein, dass im Hinblick auf die zugrundeliegende Behandlung der insbesondere neurogenen Blasenfunktionsstörungen mindestens ein weiterer Wirkstoff eingesetzt wird:
So kann es erfindungsgemäß beispielsweise vorgesehen sein, dass die Behandlung unter Verabreichung mindestens eines weiteren Wirkstoffs, insbesondere mindestens eines systemisch, vorzugsweise peroral, applizierten Wirkstoff, vorzugsweise Anticholinergikum, bevorzugt ausgewählt aus der Gruppe von Trospiumchlorid, Propiverin, Tolterodin, Fesoterodin, Darifenancin und Solifenacin sowie deren Kombinationen, insbesondere Trospiumchlorid und Propiverin sowie deren Kombinationen, erfolgt.

Erfindungsgemäß kann es in diesem Zusammenhang gleichermaßen vorgesehen sein, dass die Zusammensetzung gemeinsam und/oder in Co-Medikation mit mindestens einem insbesondere systemisch, vorzugsweise peroral, applizierten Wirkstoff, vorzugsweise Anticholinergikum, bevorzugt ausgewählt aus der Gruppe von Trospiumchlorid, Propiverin, Tolterodin, Fesoterodin, Darifenancin und Solifenacin sowie deren Kombinationen, insbesondere Trospiumchlorid und Propiverin sowie deren Kombinationen, verabreicht wird.

Hierdurch kann zum einen die Wirkeffizienz erhöht und die Behandlungsdosis von Oxybutynin, bevorzugt Oxybutyninhydrochlorid, weiterführend optimiert bzw. eingestellt werden.

Erfindungsgemäß verhält es sich insbesondere derart, dass die insbesondere neurogenen Blasenfunktionsstörungen mit einer Detrusorüberaktivität und/oder Detrusor-Sphinkter-Dyssynergie, insbesondere mit überaktivem Detrusor, einhergehen bzw. hierdurch gekennzeichnet sind.

Diesbezüglich kann es sich erfindungsgemäß gleichermaßen derart verhalten, dass die insbesondere neurogenen Blasenfunktionsstörungen mit einer das Zentralnervensystem und/oder das periphere Nervensystem betreffenden Erkrankung und/oder Verletzung und/oder Schädigung im Zusammenhang stehen oder hierdurch verursacht sind.

Diesbezüglich können die insbesondere neurogenen Blasenfunktionsstörungen mit einer Rückenmarksverletzung, insbesondere Querschnittlähmung; Spina bifida, insbesondere Meningozele, Meningomyelozele, Myelozystozele und/oder Meningomyelozystozele; Diabetes; Amyotrope Lateralsklerose; Multiple Sklerose; Hirn- und/oder Rückenmarkstumoren; Schlaganfall und/oder Morbus Parkinson im Zusammenhang stehen bzw. hierdurch verursacht sein. Vorzugsweise können die insbesondere neurogenen Blasenfunktionsstörungen mit einer Rückenmarksverletzung, insbesondere Querschnittlähmung, und/oder Spina bifida, insbesondere Meningozele, Meningomyelozele, Myelozystozele und/oder Meningomyelozystozele, im Zusammenhang stehen bzw. hierdurch verursacht sein.

Zudem kann zu der Kenngröße (ii) des zu behandelnden Patienten auch Folgendes angeführt werden:
So kann die (ii) Grunderkrankung eine das Zentralnervensystem und/oder das periphere Nervensystem betreffende Erkrankung und/oder Verletzung und/oder sein.

Zudem kann die (ii) Grunderkrankung ausgewählt sein aus der Gruppe von Rückenmarksverletzungen, insbesondere Querschnittlähmung; Spina bifida, insbesondere Meningozele, Meningomyelozele, Myelozystozele und/oder Meningomyelozystozele; Diabetes; Amyotrope Lateralsklerose; Multiple Sklerose; Hirn- und/oder Rückenmarkstumoren; Schlaganfall und Morbus Parkinson sowie deren Kombinationen. Vorzugsweise kann die Grunderkrankung ausgewählt sein aus der Gruppe von Rückenmarksverletzungen, insbesondere Querschnittlähmung, Spina bifida, insbesondere Meningozele, Meningomyelozele, Myelozystozele und/oder Meningomyelozystozele; sowie deren Kombinationen.

Im Allgemeinen kann die Zusammensetzung nach der Erfindung flüssig ausgebildet sein. Die Zusammensetzung kann insbesondere als wässrige Zusammensetzung vorliegen. Insbesondere kann die Zusammensetzung wässrig basiert bzw. ausgebildet sein, insbesondere in Form einer wässrigen Lösung. Diesbezüglich kann die Zusammensetzung Wasser, insbesondere gereinigtes Wasser, vorzugsweise Wasser für Injektionszwecke, enthalten. Insbesondere kann die Zusammensetzung Wasser als pharmazeutisch verträglichen Träger (Exzipienten) enthalten. Die Zusammensetzung kann beispielsweise einen Gehalt an Wasser von mindestens 50 Gew.-%, insbesondere mindestens 75 Gew.-%, vorzugsweise mindestens 80 Gew.-%, bezogen auf die Zusammensetzung, aufweisen. Erfindungsgemäß kann es zudem vorgesehen sein, dass die Zusammensetzung zumindest im Wesentlichen aus den Komponenten bzw. Bestandteilen in Form des Oxybutynins, insbesondere des Oxybutyninhydrochlorids, des Elektrolytsalzes, bevorzugt Natriumchlorid und Wasser, insbesondere gereinigtes Wasser, vorzugsweise Wasser für Injektionszwecke, besteht.

Im Allgemeinen kann die Zusammensetzung nach der Erfindung im Rahmen der vorliegenden Erfindung als pharmazeutische Zusammensetzung und/oder Pharmazeutikum, Arzneimittel und/oder Medikament, Medizinprodukt, Homöopathikum oder dergleichen verabreicht werden oder hergerichtet sein.

Im Allgemeinen eignet sich die Zusammensetzung nach der Erfindung zur topischen Applikation, insbesondere Instillation in die Harnblase. In diesem Zusammenhang verhält es sich erfindungsgemäß insbesondere derart, dass die Zusammensetzung zur topischen Applikation, insbesondere Instillation, in die Harnblase hergerichtet ist. Insbesondere kann die Zusammensetzung mittels topischer Applikation, insbesondere Instillation, in die Harnblase verabreicht werden.

Erfindungsgemäß kann es zudem vorgesehen sein, dass die Zusammensetzung sterilisiert, insbesondere dampfsterilisiert, bevorzugt wasserdampfsterilisiert, ist. Hierdurch wird die Anwendungssicherheit erhöht.

Dabei kann es erfindungsgemäß vorgesehen sein, dass die sterilisierte Zusammensetzung einen SAL-Wert (*Sterility Assurance Level*) von mindestens 10⁻⁵, insbesondere mindestens 10⁻⁶, vorzugsweise mindestens 10⁻⁷, aufweist bzw. dass die sterilisierte Zusammensetzung einen Gehalt an Abbauprodukt(en) des Oxybutynins, insbesondere einen Gehalt an Phenylcyclohexylglykolsäure, von höchstens 10 Gew.-%, insbesondere höchstens 7,5 Gew.-%, vorzugsweise höchstens 5 Gew.-%, bevorzugt höchstens 2 Gew.-%, besonders bevorzugt höchstens 1 Gew.-%, bezogen auf Oxybutyninhydrochlorid, aufweist. Der geringe Gehalt der Abbauprodukte bzw. die hohe Stabilität der erfindungsgemäßen Zusammensetzung auch nach Sterilisation kann insbesondere durch ein spezielles Material für die Aufbewahrungs- bzw. Applikationsvorrichtung, insbesondere in Form einer Kolbenspritze, erreicht werden, in welche die erfindungsgemäße Zusammensetzung eingebracht vorliegen kann, wie nachfolgend ausgeführt.

Im Allgemeinen kann die Zusammensetzung insbesondere anwendungs-, dosier- und/oder applikationsfertig, in einer Aufbewahrungs- und/oder Applikationsvorrichtung, vorzugsweise Kolbenspritze, bevorzugt Einweg-Kolbenspritze, insbesondere zur topischen Applikation, insbesondere Instillation, in die Harnblase, vorliegen und/oder eingebracht sein.

In diesem Zusammenhang kann die Aufbewahrungs- und/oder Applikationsvorrichtung, vorzugsweise die Kolbenspritze, bevorzugt die Einweg-Kolbenspritze, ein Volumen, insbesondere ein Aufnahmevolumen für die Zusammensetzung, im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, aufweisen.

Erfindungsgemäß kann es grundsätzlich vorgesehen sein, dass die Aufbewahrungs- und/oder Applikationsvorrichtung, vorzugsweise die Kolbenspritze, bevorzugt die Einweg-Kolbenspritze, die Zusammensetzung in einer Menge im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, aufweist.

Im Rahmen der vorliegenden Erfindung kann es zudem vorgesehen sein, dass die Aufbewahrungs- und/oder Applikationsvorrichtung, vorzugsweise die Kolbenspritze, bevorzugt die Einweg-Kolbenspritze, sterilisiert, insbesondere dampfsterilisiert, bevorzugt wasserdampfsterilisiert, ist, bevorzugt mitsamt der in der Aufbewahrungs- und/oder Applikationsvorrichtung vorliegenden und/oder eingebrachten Zusammensetzung.

Im Allgemeinen kann die Sterilisation im Rahmen der vorliegenden Erfindung bei Temperaturen im Bereich von 105 °C bis 160 °C, insbesondere im Bereich von 110 °C bis 150 °C, vorzugsweise im Bereich von 115 °C bis 140 °C, bevorzugt im Bereich von 120 °C bis 130° C, besonders bevorzugt bei etwa 121 °C, durchgeführt sein.

Zudem kann die Sterilisation für einen Zeitraum im Bereich von 1 min bis 500 min, insbesondere im Bereich von 2 min bis 400 min, vorzugsweise im Bereich von 3 min bis 200 min, bevorzugt im Bereich von 5 min bis 100 min, besonders bevorzugt im Bereich von 8 min bis 60 min, ganz besonders bevorzugt im Bereich von 10 min bis 45 min, weiter bevorzugt im Bereich von 15 min bis 30 min, noch weiter bevorzugt im Bereich von 17 min bis 25 min, nochmals weiter bevorzugt für etwa 20 min, durchgeführt sein.

Weiterhin kann die Dampfsterilisation unter Druckbeaufschlagung durchgeführt sein.

Insbesondere kann die Sterilisation als Dampfsterilisation gemäß Pharmacopoea Europaea (Ph. Eur.), Kapitel 5.1.1 (07/2017:50101) durchgeführt sein, insbesondere unter *overkill*-Bedingungen und/oder bei etwa 121 °C und/oder für einen Zeitraum von mindestens 15 min, insbesondere von mindestens 20 min, vorzugsweise von etwa 20 min. Diesbezüglich kann auch auf die weiteren Ausführungen gemäß Pharmacopea Europaea (Ph. Eur.), Kapitel 5.1.1 (07/2017:50101) verwiesen werden.

Die im Hinblick auf die erfindungsgemäße Zusammensetzung eingesetzte Aufbewahrungs- bzw. Applikationsvorrichtung, welche insbesondere in Form einer Kolbenspritze, bevorzugt Einweg-Kolbenspritze vorliegt bzw. ausgebildet ist, kann im Rahmen der vorliegenden Erfindung insbesondere aus einem Cycloolefin-(Co-)Polymer (COP bzw. COC) gebildet sein.

Dabei kann es insbesondere vorgesehen sein, dass die Aufbewahrungs- und/oder Applikationsvorrichtung als Kolbenspritze, bevorzugt Einweg-Kolbenspritze, ausgebildet ist und/oder in Form einer Kolbenspritze, bevorzugt Einweg-Kolbenspritze, vorliegt.

Diesbezüglich kann die Kolbenspritze einen Spritzenkörper, insbesondere Spritzenzylinder, einerseits und einen Spritzenkolben mit einem Kolbenstopfen andererseits aufweisen, wobei der zugrundeliegende Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist.

Insbesondere kann es erfindungsgemäß vorgesehen sein, dass die Aufbewahrungs- und/oder Applikationsvorrichtung als Kolbenspritze, bevorzugt Einweg-Kolbenspritze, ausgebildet ist und/oder in Form einer Kolbenspritze, bevorzugt Einweg-Kolbenspritze, vorliegt, insbesondere wobei die Kolbenspritze, insbesondere der Spritzenzylinder der Kolbenspritze, eine erste Öffnung, insbesondere zur Aufnahme eines Spritzenkolbens, aufweist; und/oder insbesondere wobei die Kolbenspritze einen Spritzenkolben aufweist, insbesondere wobei der Spritzenkolben aus Polypropylen gebildet ist oder insbesondere wobei der Spritzenkolben aus einem Cycloolefin-(Co-)Polymer (COP bzw. COC) gebildet ist.

Zudem kann es erfindungsgemäß vorgesehen sein, dass die Aufbewahrungs- und/oder Applikationsvorrichtung als Kolbenspritze, bevorzugt Einweg-Kolbenspritze, ausgebildet ist und/oder in Form einer Kolbenspritze, bevorzugt Einweg-Kolbenspritze, vorliegt, insbesondere wobei die Kolbenspritze eine zweite Öffnung, insbesondere Austragsöffnung, vorzugsweise zum Austragen und/oder Applizieren der oxybutyninhaltigen Zusammensetzung, aufweist bzw. insbesondere wobei die Kolbenspritze ein Verschlusselement, insbesondere eine Verschlusskappe, vorzugsweise zum Verschließen der zweiten Öffnung, aufweist, insbesondere wobei das Verschlusselement aus einem Halogenbutylkautschuk, insbesondere aus einem Chlorbutylkautschuk oder aus einem Brombutylkautschuk, vorzugsweise aus einem Brombutylkautschuk, gebildet ist.

Durch die Verwendung der zuvor angeführten Materialien bzw. Polymere für die Aufbewahrungs- bzw. Applikationsvorrichtung wird insbesondere eine hohe Kompatibilität gegenüber der erfindungsgemäßen Zusammensetzung auch im Rahmen der zugrundeliegenden Sterilisation gewährleistet, was zu entsprechend verbesserten Eigenschaften der diesbezüglich sterilisierten oxybutyninhaltigen Zusammensetzung nach der Erfindung führt, und zwar auch im Hinblick auf eine Verringerung des Gehalts an unerwünschten Abbauprodukten.

Wie zuvor angeführt, wird durch die Verwendung der in Rede stehenden Polymere eine hohe Kompatibilität im Rahmen der erfindungsgemäß eingesetzten Dampfsterilisation gewährleistet, was zu entsprechend verbesserten Eigenschaften der sterilisierten oxybutyninhaltigen Zusammensetzung führt. Im Hinblick auf die erfindungsgemäß eingesetzten Cycloolefin-(Co-)Polymere (COP bzw. COC) können markt- bzw. handelsübliche Produkte verwendet werden (wie beispielsweise TopPAcPolymer Topas COC Grade 6013B-51; Grade EuP 3.2.8).

Was die eingesetzten Materialien auf Basis von Halogenbutylkautschuk, insbesondere Brombutylkautschuk anbelangt, so können diesbezüglich gleichermaßen markt- bzw. handelsübliche Produkte eingesetzt werden (wie z. B. Dätwyler V9340, FM257/2 Bromobutyl-Compound).

Gemäß dem vorliegenden Aspekt betrifft die vorliegende Erfindung auch eine oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird, insbesondere eine wie zuvor beschriebene Zusammensetzung,
wobei die Zusammensetzung das Oxybutynin bevorzugt in Form des Hydrochlorids enthält, die Zusammensetzung als wässrige Zusammensetzung ausgebildet ist und die Zusammensetzung in Kombination und jeweils in wirksamen, insbesondere pharmazeutisch wirksamen Mengen,
- Oxybutynin, bevorzugt Oxybutyninhydrochlorid (Oxybutynin-HCl), in einer Konzentration von (1 ± 0,5) mg/ml und
- mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,5) mg/ml
enthält,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder mit der Maßgabe und/oder Zielsetzung, dass der Detrusorkoeffizient (*Compliance*) unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

Die vorliegende Erfindung betrifft gemäß dem vorliegenden Aspekt auch eine oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird, insbesondere eine wie zuvor beschriebene Zusammensetzung,
wobei die Zusammensetzung das Oxybutynin bevorzugt in Form des Hydrochlorids enthält, die Zusammensetzung als wässrige Zusammensetzung ausgebildet ist und die Zusammensetzung in Kombination und jeweils in wirksamen, insbesondere pharmazeutisch wirksamen Mengen,
- Oxybutynin, bevorzugt Oxybutyninhydrochlorid (Oxybutynin-HCl), in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
- mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml,
enthält,
wobei die Zusammensetzung einen pH-Wert im Bereich von 3 bis 5, insbesondere im Bereich von 3,8 bis 4,5, aufweist, insbesondere wobei der pH-Wert der Zusammensetzung unter Verwendung von und/oder mittels Salzsäure eingestellt ist,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder mit der Maßgabe und/oder Zielsetzung, dass der Detrusorkoeffizient (*Compliance*) unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

Die vorliegende Erfindung betrifft gleichermaßen gemäß dem vorliegenden Aspekt auch eine oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird, insbesondere eine wie zuvor beschriebene Zusammensetzung,
wobei die Zusammensetzung das Oxybutynin bevorzugt in Form des Hydrochlorids enthält, die Zusammensetzung als wässrige Zusammensetzung ausgebildet ist und die Zusammensetzung in Kombination und jeweils in wirksamen, insbesondere pharmazeutisch wirksamen Mengen,
- Oxybutynin, bevorzugt Oxybutyninhydrochlorid (Oxybutynin-HCl), in einer Konzentration von etwa 1 mg/ml und
- mindestens ein Elektrolytsalz, bevorzugt Natriumchlorid, berechnet als Elektrolytkation, bevorzugt berechnet als Natrium im Fall von Natriumchlorid, in einer Konzentration von etwa 3,5 mg/ml
enthält,
wobei die Zusammensetzung einen pH-Wert im Bereich von 3 bis 5, insbesondere im Bereich von 3,8 bis 4,5, aufweist, insbesondere wobei der pH-Wert der Zusammensetzung unter Verwendung von und/oder mittels Salzsäure eingestellt ist,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder mit der Maßgabe und/oder Zielsetzung, dass der Detrusorkoeffizient (*Compliance*) unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

Darüber hinaus betrifft die vorliegende Erfindung gemäß dem vorliegenden Aspekt auch eine oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird, insbesondere eine wie zuvor beschriebene Zusammensetzung,
wobei die Zusammensetzung das Oxybutynin bevorzugt in Form des Hydrochlorids enthält, die Zusammensetzung als wässrige Zusammensetzung ausgebildet ist und die Zusammensetzung in Kombination und jeweils in wirksamen, insbesondere pharmazeutisch wirksamen Mengen,
- Oxybutynin, bevorzugt Oxybutyninhydrochlorid (Oxybutynin-HCl), in einer Konzentration von etwa 1 mg/ml und
- mindestens ein Elektrolytsalz, bevorzugt Natriumchlorid, berechnet als Elektrolytkation, bevorzugt berechnet als Natrium im Fall von Natriumchlorid, in einer Konzentration von etwa 3,5 mg/ml
enthält,
wobei die Zusammensetzung einen pH-Wert im Bereich von 3 bis 5, insbesondere im Bereich von 3,8 bis 4,5, aufweist, insbesondere wobei der pH-Wert der Zusammensetzung unter Verwendung von und/oder mittels Salzsäure eingestellt ist,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder mit der Maßgabe und/oder Zielsetzung, dass der Detrusorkoeffizient (*Compliance*) unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung, und
wobei die Zusammensetzung, insbesondere anwendungs-, dosier- und/oder applikationsfertig, in einer Aufbewahrungs- und/oder Applikationsvorrichtung, vorzugsweise Kolbenspritze, bevorzugt Einweg-Kolbenspritze, insbesondere zur topischen Applikation, insbesondere Instillation, in die Harnblase, vorliegt und/oder eingebracht ist.

Die vorliegende Erfindung betrifft gemäß dem vorliegenden Aspekt auch die erfindungsgemäß definierte Zusammensetzung zur prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase.

Hinsichtlich weiterer diesbezüglicher Ausgestaltungen kann zudem auf die Ausführungen zu den weiteren erfindungsgemäßen Aspekten verwiesen werden, welche entsprechend gelten.

Die vorliegende Erfindung betrifft weiterhin - gemäß einem **weiteren** Aspekt der vorliegenden Erfindung - auch die Verwendung der erfindungsgemäßen Zusammensetzung, wie zuvor definiert, zur prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase.

In diesem Zusammenhang betrifft die vorliegende Erfindung gleichermaßen auch die Verwendung der erfindungsgemäßen Zusammensetzung, wie zuvor definiert, zur Herstellung eines Arzneimittels und/oder Medikaments zur prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase.

Für weitergehende Einzelheiten zu den erfindungsgemäßen Verwendungen der Zusammensetzung nach der Erfindung kann auch auf die Ausführungen zu den übrigen Erfindungsaspekten verwiesen werden, welche in Bezug auf die erfindungsgemäßen Verwendungen entsprechend gelten.

Gemäß einem nochmals **weiteren** Aspekt der vorliegenden Erfindung betrifft die vorliegende Erfindung auch eine Aufbewahrungs- und/oder Applikationsvorrichtung, vorzugsweise Kolbenspritze, bevorzugt Einweg-Kolbenspritze, vorzugsweise zur topischen Applikation, insbesondere Instillation, in die Harnblase, enthaltend eine oxybutyninhaltige Zusammensetzung, wie zuvor definiert.

In diesem Zusammenhang kann es sich im Rahmen der vorliegenden Erfindung insbesondere derart verhalten, dass die Aufbewahrungs- und/oder Applikationsvorrichtung, vorzugsweise die Kolbenspritze, bevorzugt die Einweg-Kolbenspritze, sterilisiert, insbesondere dampfsterilisiert, bevorzugt wasserdampfsterilisiert, ist, wie zuvor definiert, bevorzugt mitsamt bzw. einschließlich der in der Aufbewahrungs- und/oder Applikationsvorrichtung vorliegenden und/oder eingebrachten oxybutyninhaltigen Zusammensetzung nach der Erfindung bzw. wie zuvor definiert.

Im Allgemeinen kann die Aufbewahrungs- und/oder Applikationsvorrichtung, vorzugsweise die Kolbenspritze, bevorzugt die Einweg-Kolbenspritze, ein Volumen, insbesondere ein Aufnahmevolumen für die Zusammensetzung nach der Erfindung, im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, aufweisen.

Insbesondere kann die Aufbewahrungs- bzw. Applikationsvorrichtung, vorzugsweise die Kolbenspritze, bevorzugt die Einweg-Kolbenspritze, die Zusammensetzung nach der Erfindung in einer Menge im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, aufweisen.

Für weitergehende Einzelheiten zu der erfindungsgemäßen Aufbewahrungs- bzw. Applikationsvorrichtung, welche die Zusammensetzung nach der Erfindung enthält, kann zudem auf die Ausführungen zu den übrigen Erfindungsaspekten verwiesen werden, welche vorliegend entsprechend gelten.

Die vorliegende Erfindung betrifft darüber hinaus - gemäß einem wiederum **weiteren** Aspekt der vorliegenden Erfindung - auch die Verpackungseinheit nach der Erfindung, welche mindestens eine Verpackung und mindestens eine Aufbewahrungs- bzw. Applikationsvorrichtung, vorzugsweise Kolbenspritze, bevorzugt Einweg-Kolbenspritze, wie zuvor definiert, enthält, wobei die Aufbewahrungs- und/oder Applikationsvorrichtung, vorzugsweise die Kolbenspritze, bevorzugt die Einweg-Kolbenspritze, in die Verpackung eingebracht ist und/oder in der Verpackung vorliegt. In diesem Zusammenhang ist es erfindungsgemäß insbesondere vorgesehen, dass die erfindungsgemäße Zusammensetzung, wie zuvor definiert, gleichermaßen in der Aufbewahrungs- bzw. Applikationsvorrichtung eingebracht ist. Für weitergehende Einzelheiten zu der erfindungsgemäßen Verpackungseinheit kann auch auf die Ausführungen zu den übrigen Aspekten der vorliegenden Erfindung verwiesen werden, welche in Bezug auf die erfindungsgemäße Verpackungseinheit in entsprechender Weise gelten.

Die vorliegende Erfindung betrifft - gemäß einem **weiteren** Aspekt der vorliegenden Erfindung - auch das erfindungsgemäße Kit, insbesondere Applikations- und/oder Instillationssystem, umfassend (I) mindestens eine in einer Aufbewahrungs- und/oder Applikationsvorrichtung, vorzugsweise Kolbenspritze, bevorzugt Einweg-Kolbenspritze, insbesondere wie zuvor definiert, vorliegende oxybutyninhaltige Zusammensetzung, wie zuvor definiert; (II) (a) mindestens eine an die Aufbewahrungs- und/oder Applikationsvorrichtung, vorzugsweise Kolbenspritze, bevorzugt Einweg-Kolbenspritze, anschließbare Applikations- und/oder Instillationsvorrichtung, insbesondere in Form eines Instillationsschlauches oder dergleichen, vorzugsweise in Form eines (Harnblasen-)Katheters, und/oder (b) mindestens eine an die Aufbewahrungs- und/oder Applikationsvorrichtung, vorzugsweise Kolbenspritze, bevorzugt Einweg-Kolbenspritze, anschließbare Anschlussvorrichtung, insbesondere Adapter, vorzugsweise zum Anschließen an die Applikations- und/oder Instillationsvorrichtung, insbesondere in Form eines Instillationsschlauches oder dergleichen, vorzugsweise in Form eines (Harnblasen-)Katheters; und gegebenenfalls (III) mindestens eine Applikations- und/oder Instillationsanleitung.

In diesem Zusammenhang betrifft die vorliegende Erfindung auch das erfindungsgemäße Kit, insbesondere Applikations- und/oder Instillationssystem, umfassend (I) mindestens eine Kolbenspritze, insbesondere Einweg-Kolbenspritze, insbesondere wie zuvor definiert, und/oder mindestens eine Verpackungseinheit, wie zuvor definiert; (II) (a) mindestens eine an die Kolbenspritze anschließbare Applikations- und/oder Instillationsvorrichtung, insbesondere in Form eines Instillationsschlauches oder dergleichen, vorzugsweise in Form eines (Harnblasen-)Katheters, und/oder (b) mindestens eine an die Kolbenspritze anschließbare Anschlussvorrichtung, insbesondere Adapter, zum Anschließen an eine Applikations- und/oder Instillationsvorrichtung, insbesondere in Form eines Instillationsschlauches oder dergleichen, vorzugsweise in Form eines (Harnblasen-)Katheters; und gegebenenfalls (III) mindestens eine Applikations- und/oder Instillationsanleitung.

Auf Basis des erfindungsgemäßen Kits wird insbesondere ein entsprechendes Instillationssystem bereitgestellt, welches bei einfacher Handhabbarkeit eine schnelle Einsatzbereitschaft unter hoher Anwendungssicherheit aufweist.

Für weitergehende Einzelheiten zu dem erfindungsgemäßen Kit kann auch auf die Ausführungen zu den übrigen Erfindungsaspekten verwiesen werden, welche vorliegend entsprechend gelten.

Darüber hinaus betrifft die vorliegende Erfindung - gemäß einem wiederum **weiteren** Aspekt der vorliegenden Erfindung - auch das Verfahren zur Bestimmung und/oder Festlegung einer Behandlungsdosis, insbesondere einer tagesbezogenen Therapiedosis, von Oxybutynin, insbesondere Oxybutyninhydrochlorid, als Wirkstoff bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, unter Verwendung einer oxybutyninhaltigen Zusammensetzung, insbesondere wie zuvor definiert, wobei die Zusammensetzung mittels topischer Verabreichung, insbesondere mittels Instillation, in die Harnblase appliziert wird,
wobei die Zusammensetzung das Oxybutynin bevorzugt in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutynin, bevorzugt Oxybutyninhydrochlorid (Oxybutynin-HCl), in einer Konzentration von (1 ± 0,5) mg/ml und
- mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,5) mg/ml
enthält,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion.

Für weitergehende Einzelheiten zu dem erfindungsgemäßen Verfahren kann auch auf die Ausführungen zu den übrigen Erfindungsaspekten verwiesen werden, welche in Bezug auf das erfindungsgemäße Verfahren entsprechend gelten.

Im Rahmen der vorliegenden Erfindung wird somit insgesamt unter Zugrundelegung der erfindungsgemäßen Zusammensetzung ein leistungsfähiges Gesamtkonzept insbesondere im Hinblick auf eine optimierte Bestimmung und Festlegung einer Behandlungsdosis der Wirksubstanz Oxybutynin bzw. Oxybutyninhydrochlorid bei der Behandlung von insbesondere neurogenen Blasenfunktionsstörungen unter Instillation der diesbezüglichen Zusammensetzung bereitgestellt, was zu einer optimierten Einstellung der diesbezüglich relevanten urodynamischen Kenngrößen mit einer Verbesserung der Harnblasenfunktion insgesamt führt.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

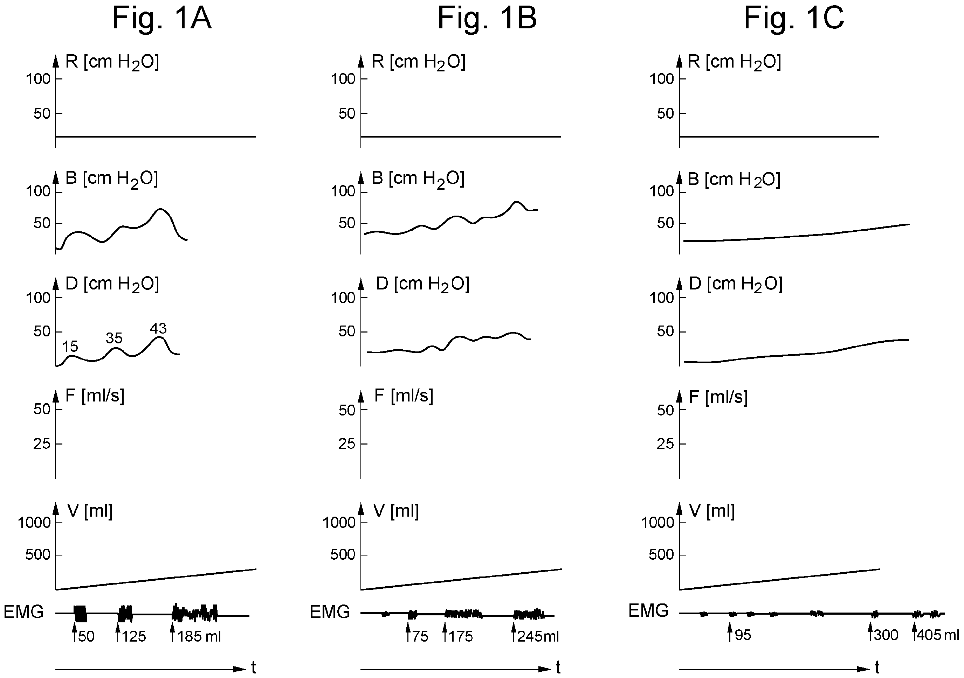

Die vorliegende Erfindung wird nachfolgend in den Ausführungsbeispielen auch anhand von Ausführungsformen bzw. Ausgestaltungen der vorliegenden Erfindung darstellenden Figurendarstellung beschrieben (vgl. Fig. 1A, 1B, 1C; Fig. 2A, 2B, 2C; Fig. 3A, 3B, 3C sowie Fig. 4A, 4B, 4C und Fig. 5A, 5B, 5C). Im Zusammenhang mit der Erläuterung dieser Ausführungsformen bzw. Ausgestaltungen der vorliegenden Erfindung, welche jedoch in Bezug auf die vorliegende Erfindung keinesfalls beschränkend sind, werden auch weitergehende Vorteile, Eigenschaften, Aspekte und Merkmale der vorliegenden Erfindung aufgezeigt.

Die folgenden Ausführungsbeispiele dienen dabei lediglich der Veranschaulichung der vorliegenden Erfindung, jedoch ohne die vorliegende Erfindung hierauf zu beschränken.

### AUSFÜHRUNGSBEISPIELE:

Nachfolgend werden Fallbeispiele in Form von Fallberichten angeführt, welche das erfindungsgemäße Therapie- bzw. Dosisfindungskonzept zur Bestimmung der Behandlungsdosis bzw. tagesbezogenen Therapiedosis von Oxybutynin bzw. Oxybutyninhydrochlorid bei zugrundeliegender neurogener Blasenfunktionsstörung aufzeigen. Die zugrundeliegenden Patienten werden anamnetisch erfasst und die urodynamischen Parameter bzw. Kenngrößen unter Heranziehung einer entsprechenden Urodynamik erfasst bzw. bestimmt. Für sämtliche Patienten gilt, dass unter Anwendung der erfindungsgemäßen Zusammensetzung mit der diesbezüglichen Dosisfindung eine Verbesserung auch des Zielparameters in Form des Detrusordrucks vorliegt. Dabei umfassen die Fallbeispiele auch solche unter die erfindungsgemäße Konzeption fallende Aspekte, bei welchen gegebenenfalls neben dem mittels Instillation in die Harnblase verabreichten Oxybutynin bzw. Oxybutyninhydrochlorid gegebenenfalls noch weitere anticholinerge Wirkstoffe, welche systemisch bzw. peroral verabreicht werden, im Rahmen einer Co-Therapie hinzugezogen werden können.

Zu den Fallberichten im Einzelnen:

### 1. Fallbericht I:

### Patientin mit Multipler Sklerose als neurogene Grunderkrankung

Eine Patientin (Alter: 44 Jahre) mit Multipler Sklerose vom schubförmigen Verlauf leidet seit dem letzten Schubereignis vor acht Monaten unter starkem Harndrang mit Toilettengängen im Stundentakt tagsüber sowie mindestens dreimal nachts, wobei zudem eine regelmäßige Dranginkontinenz (nOABwet) vorliegt und wobei mindestens vier große Vorlagen tagsüber und ein bis zwei Schutzhosen in der Nacht als aufsaugende Schutzmittel benötigt werden.

Die Patientin wird initial mit Fesoterodin (3,5 mg Tablette 1 x 1/d) zur Blasendämpfung behandelt, dies jedoch ohne jeglichen Erfolg. Anschließend erhält die Patientin seit nunmehr acht Wochen Trospiumchlorid (3 x 15 mg / d oral). Hierunter ist allenfalls eine geringgradige Linderung mit diskreter Reduktion der Miktionsfrequenz und unveränderter Dranginkontinenz zu beobachten. Jedoch treten anticholinerge Nebenwirkungen mit Mundtrockenheit, Verstopfungsneigung und Akkomodationsstörung mit verschwommenem Sehen auf.

Die Urodynamik unter 3 x 15 mg Trospiumchlorid zeigt dabei folgenden Befund:

| | |
|---|---|
| erster Harndrang / korrespondierender Detrusordruck: | 50 ml / 15 cm H₂O |
| starker Harndrang / korrespondierender Detrusordruck: | 125 ml / 35 cm H₂O |
| maximales Füllvolumen / korrespondierender Detrusordruck: | 185 ml / 43 cm H₂O |
| Detrusorinstabilität ab: | 15 ml |
| max. Detrusordruck während Füllung: | 43 cm H₂O |

Hierzu kann auch auf die Figurendarstellung gemäß Fig. 1A verwiesen werden (mit R = Rektum, B = Blase, D = Detrusor, F = *Flow* bzw. Flussrate bei Miktion, V = Volumen und EMG = Elektromyogramm).

Zusammenfassend liegt somit eine neurogene Blasenfunktionsstörung im Sinne einer hypersensitiven, hypokapazitären, instabilen und hypertonen Harnblase vor.

Im Folgenden wird daher eine Therapieeskalation auf Instillation von flüssigem Oxybutynin bzw. Oxybutyninhydrochlorid (2 x 10 ml / d, entspricht 2 x 10 mg/d) durchgeführt, wobei die orale Einnahme von Trospiumchlorid abgesetzt wird. Hierunter ist eine deutliche Symptomlinderung mit Reduktion der Miktionsfrequenz auf acht Toilettengänge tagsüber und ein bis zwei Toilettengänge nachts zu beobachten. Zudem liegt eine Reduktion der Dranginkontinenzepisoden / Ereignisse sowie des Vorlagenverbrauchs um 50 % vor, wobei nur noch eine Schutzhose pro Nacht benötigt wird. Zudem liegen keine anticholinergen Nebenwirkungen mehr vor.

Die Kontroll-Urodynamik unter 2 x 10 mg flüssigem Oxybutynin zeigt:

| | |
|---|---|
| erster Harndrang / korrespondierender Detrusordruck: | 75 ml / 15 cm H₂O |
| starker Harndrang / korrespondierender Detrusordruck: | 175 ml / 28 cm H₂O |
| maximales Füllvolumen / korrespondierender Detrusordruck: | 245 ml / 33 cm H₂O |
| Detrusorinstabilität ab: | 120 ml |
| max. Detrusordruck während Füllung: | 36 cm H₂O |

Hierzu kann auch auf Fig. 1B verwiesen werden (wobei zu den diesbezüglichen Abkürzungen auf die Ausführungen zu Fig. 1A verwiesen werden kann).

Zusammenfassend liegt somit eine neurogene Blasenfunktionsstörung im Sinne einer hypersensitiven, hypokapazitären, instabilen, normotonen Harnblase vor.

Die potentiell nierengefährdenden Detrusordrücke können zudem unter 40 cm H₂O gesenkt werden, die Blase ist jedoch immer noch hypokapazitär. Die Patientin ist nahezu unverändert, jedoch geringgradiger dranginkontinent, so dass eine Verdoppelung der Oxybutynindosis auf 4 x 10 mg erfolgt. Hierunter kann eine weitere Reduktion der Miktionsfrequenz auf sechs Toilettengänge pro Tag und auf einen Toilettengang pro Nacht sowie eine nur noch gelegentliche Dranginkontinenz tagsüber, vor allem in Stresssituationen (Reiseantritt mit unsicherer Toilettenversorgung, familiäre Belastung / Sorge) beobachtet werden.

Die Verlaufs-Urodynamik nach sechs Monaten 4 x 10 mg flüssigem Oxybutynin zeigt:

| | |
|---|---|
| erster Harndrang / korrespondierender Detrusordruck: | 95 ml / 5 cm H₂O |
| starker Harndrang / korrespondierender Detrusordruck: | 295 ml / 14 cm H₂O |
| maximales Füllvolumen / korrespondierender Detrusordruck: | 405 ml / 24 cm H₂O |
| Detrusorinstabilität ab: | 400 ml |
| max. Detrusordruck während Füllung: | 24 cm H₂O |

Hierzu kann auch auf Fig. 1C verwiesen werden (wobei zu den diesbezüglichen Abkürzungen auf die Ausführungen zu Fig. 1A verwiesen werden kann).

Zusammenfassend liegt somit abschließend eine neurogene Blasenfunktionsstörung im Sinne einer normosensitiven, normakapazitären, terminal diskret instabilen, normotonen Harnblase vor, einhergehend mit einer entsprechenden Verbesserung der diesbezüglichen Zielparameter in Form des Detrusordrucks.

### 2. Fallbericht II:

### Patient mit hoher Querschnittlähmung subC2 als neurogene Grunderkrankung

Bei einem 21-jährigen Patienten mit Zustand nach Motorradunfall und u.a. Bruch des zweiten Halswirbelkörpers liegt eine komplette motorische und sensorische Querschnittlähmung unterhalb des zweiten Halswirbelkörpers mit muskulären Spastiken aller Extremitäten (Tetraspastik) vor. Der Patient ist dauerhaft über ein Tracheostoma (Beatmungskanüle über Luftröhrenschnitt) beatmungspflichtig. Die Harnblase wird viermal tagsüber sowie einmal nachts durch Familienangehörige oder Pflegepersonal fremdkatheterisiert. Mit Austritt aus dem sogenannten spinalen Schock entwickelt sich ab der sechsten Woche nach Unfallereignis eine Harndranginkontinenz, die in einer ersten Urodynamik zu diesem Zeitpunkt ein messtechnisches Korrelat in Form von Detrusorüberaktivitäten mit maximalen Drücken von 50 cm H₂O und einem *leak point pressure* von 48 cm H₂O aufweist.

Daraufhin erfolgt eine Detrusordämpfung mit Trospiumchlorid (3 x 30 ml /d oral). Da hierunter die Harninkontinenz jedoch sistiert und im Weiteren zusätzlich die Katheterisierungshäufigkeit auf sechsmal pro Tag und zweimal pro Nacht gesteigert werden muss, da andernfalls bei höheren Blasenfüllungsvolumina Symptome einer vegetativen Dysregulation auftreten (Kopfschmerz, Rötung der Gesichtshaut / des Dekolletes sowie Gänsehaut) wird nach zwei Wochen zusätzlich Oxybutynin (3 x 5 mg / d oral) verabreicht. Hierunter wird eine Kontrolldynamik nach einer Woche mit der oben genannten oralen Kombinationstherapie und insgesamt in Woche zehn nach Unfall durchgeführt.

Die Urodynamik unter Tagesdosis von 3 x 30 mg Trospiumchlorid und 3 x 5 mg Oxybutynin zeigt:

| | |
|---|---|
| erster Harndrang / korrespondierender Detrusordruck: | - / - (komplette Tetraplegie ohne Füllungsempfinden) |
| starker Harndrang / korrespondierender Detrusordruck: | - / - |
| maximales Füllvolumen / korrespondierender Detrusordruck: | Abbruch bei 285 ml / 65 cm H₂O infolge von Kopfschmerz und Gänsehaut sowie Blutdruckanstieg auf 195 / 110 mm Hg (vegetative Dysreflexie) |
| Detrusorinstabilität ab: | 20 ml |
| max. Detrusordruck während Füllung: | 66 cm H₂O |

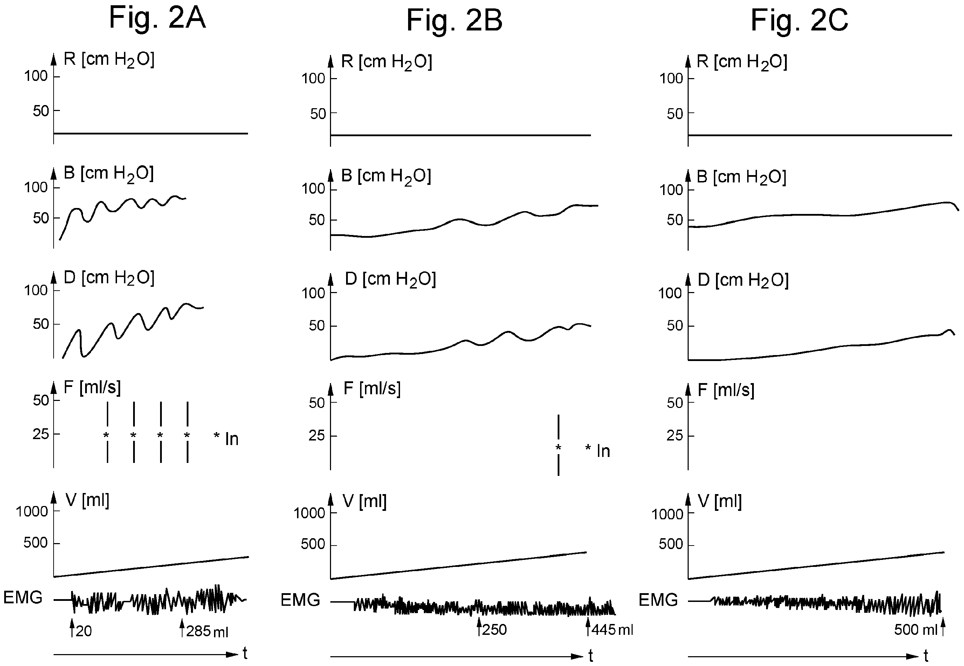

Hierzu kann auch auf Fig. 2A verwiesen werden (wobei zu den diesbezüglichen Abkürzungen auf die Ausführungen zu Fig. 1A verwiesen werden kann sowie mit In = Inkontinenz).

Zusammenfassend liegt somit eine neurogene Blasenfunktionsstörung im Sinne einer asensitiven, hypokapazitären, instabilen, hypertonen Harnblase vor.

Im Folgenden wird daher eine Therapieeskalation auf zusätzliche Instillation von flüssigem Oxybutynin bzw. Oxybutyninhydrochlorid (3x10ml/d, entspricht 3 x 10 mg / d) durchgeführt. Hierunter ist eine Reduktion der Katheterisierungsfrequenz auf 6 x / 24 h und ein Anstieg des funktionellen Blasenvolumens auf 350 ml bis 400 ml / Katheterisierung und ein Rückgang der Inkontinenzereignisse auf etwa 1 x / d zu beobachten.

Die Kontroll-Urodynamik in Woche 14 nach Unfallereignis unter zusätzlich 3 x 10 mg flüssigem Oxybutynin zeigt:

| | |
|---|---|
| maximales Füllvolumen / korrespondierender Detrusordruck: | Abbruch bei 245 ml / 55 cm H₂O, dann RR-Anstieg auf 175 / 95 mm Hg |
| Detrusorinstabilität ab: | 250 ml |
| max. Detrusordruck während Füllung: | 55 cm H₂O |

Hierzu kann auch auf Fig. 2B verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer asensitiven, normo- bis hypokapazitären, instabilen, hypertonen Harnblase vor.

Da die Detrusordrücke 40 cm H₂O überschreiten und im klinischen Alltag als auch unter Messung unverändert Harninkontinenz und Symptome einer vegetativen Dysreflexie auftreten, wird die Oxybutynindosis auf 6 x 10 mg / d gesteigert. Hierunter liegt ein Sistieren der Dranginkontinenz und der Symptome einer vegetativen Dysregulation vor.

Die Verlaufs-Urodynamik nach sechs Monaten 3 x 5 mg oralem Oxybutynin und zusätzlich 6 x 10 mg flüssigem Oxybutynin zeigt:

| | |
|---|---|
| maximales Füllvolumen / korrespondierender Detrusordruck: | 500 ml / 40 cm H₂O |
| Detrusorinstabilität ab: | 475 ml |
| max. Detrusordruck während Füllung: | 40 cm H₂O |

Hierzu kann auch auf Fig. 2C verwiesen werden (wobei zu den diesbezüglichen Abkürzungen auf die Ausführungen zu Fig. 1A bzw. Fig. 2A verwiesen werden kann).

Zusammenfassend liegt somit abschließend eine neurogene Blasenfunktionsstörung im Sinne einer asensitiven, normokapazitären, ultraterminal instabilen, grenzwertig normotonen Harnblase vor.

### 3. Fallbericht III:

### Patientin mit Spina bifida (Meningomyelocele, MMC) als neurogene Grunderkrankung

Bei einem zwölfjährigen Mädchen mit lumbaler MMC, bei welcher bislang keine neuro-urologische Versorgung / Mitbetreuung durchgeführt worden ist, liegt eine Rollstuhlpflichtigkeit vor. Zudem liegen eine alltägliche Harninkontinenz, eine Verstopfungsneigung sowie mindestens einmal pro Quartal zum Teil fieberhafte Harnwegsinfekte mit einer Antibiotikatherapie (hierunter dann typischerweise Stuhlinkontinenz) vor. Zudem erfolgt sowohl tagsüber als auch nachts eine Windelversorgung. Eine erste Videourodynamik zeigt eine Harnblase mit einer *low compliance* mit sofortigen Detrusordruckanstiegen mit Füllungsbeginn und raschem Überschreiten der Detrusordrücke von 40 cm H₂O, wobei der maximale Detrusordruck zum Füllstopp bei 210 ml 115 cm H₂O beträgt. Zu diesem Zeitpunkt wird in der Röntgendurchleuchtung ein beidseitiger Reflux von Kontrastmittel in beide Harnleiter bis ins Nierenbecken beobachtet, wobei das ableitende Hohlsystem geringgradig aufballoniert bzw. erweitert ist (= Reflux dritten Grades). Eine Nierenszintigrafie zeigt eine gerade noch normwertige Nierenfunktionsleistung mit symmetrischer Leistungsverteilung auf beide Nieren. Zudem liegt ein durchgehend tonisch-klonisches Beckenboden-EMG-Signal als Ausdruck einer Beckenbodenspastik / Beckenboden-Relaxationsstörung mit resultierender Restharnbildung in Höhe von 65 ml vor.

Es wird eine Blasendämpfung mit Propiverin (3 x 15 ml / d oral) sowie ein intermittierender Fremd- und Selbstkatheterismus eingeleitet. Hierunter resultieren im Alltag Kathetervolumina von 200 ml, eine Reduktion der Inkontinenzepisoden und -volumina und ein Wechsel der Windelversorgung auf ca. zwei bis drei Vorlagen tagsüber. Allerdings liegen anticholinerge Nebenwirkungen vor, welche die Patientin bzw. die Familie der Patientin als inakzeptabel beschreibt. Hierzu zählen schwere Mundtrockenheit, trockene Kehle, Akkommodationsstörungen, Kopfschmerzen, Konzentrationsstörungen sowie eine dauerhafte Obstipation.

Die erste Kontroll-Urodynamik unter Tagesdosis von 3 x 15 mg Propiverin zeigt:

| | |
|---|---|
| erster Harndrang / korrespondierender Detrusordruck: | sofort mit Füllungsbeginn |
| starker Harndrang / korrespondierender Detrusordruck: | - / - |
| maximales Füllvolumen / korrespondierender Detrusordruck: | Abbruch bei 250 ml / 85 cm H₂O infolge kontinuierlicher Inkontinenz am Messkatheter entlang |
| Detrusorinstabilität ab: | keine, *low compliance* von 3 ml / cm H₂O mit linearem Druckanstieg |
| max. Detrusordruck während Füllung: | 85 cm H₂O |

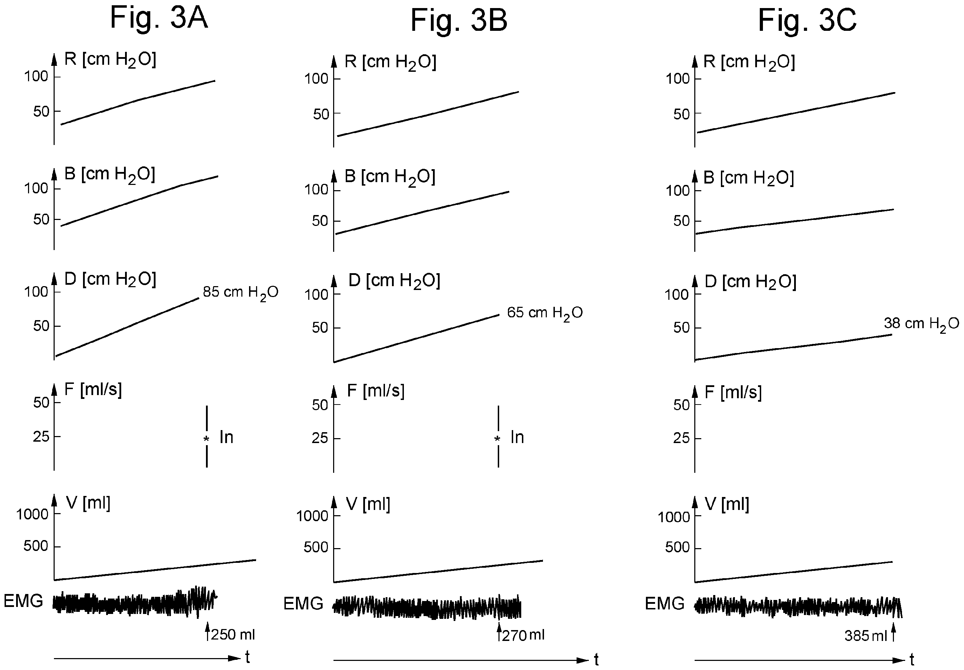

Hierzu kann auch auf Fig. 3A verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hypersensitiven, hypokapazitären, hypertonen, *low-compliance* Harnblase vor.

Im Folgenden wird daher ein Therapiewechsel bzw. eine Therapieadaptation auf Instillation von flüssigem Oxybutynin bzw. Oxybutyninhydrochlorid (3 x 8 ml / d entsprechend 3 x 8 mg / d) durchgeführt. Hierunter kann eine weitere Reduktion der Inkontinenzereignisse und -volumina auf etwa einmal pro Tag und einmal pro Nacht sowie ein Anstieg des funktionellen Blasenvolumens auf 250 ml beobachtet werden.

Die Kontroll-Urodynamik unter 3 x 8 mg flüssigem Oxybutynin zeigt:

| | |
|---|---|
| erster Harndrang / korrespondierender Detrusordruck: | 40 ml / 10 cm H₂O |
| starker Harndrang / korrespondierender Detrusordruck: | - / - |
| maximales Füllvolumen / korrespondierender Detrusordruck: | Abbruch bei 270 ml / 65 cm H₂O infolge kontinuierlicher Inkontinenz am Messkatheter entlang |
| Detrusorinstabilität ab: | Keine, *low compliance* von 4,2 ml / cm H₂O mit linearem Druckanstieg |
| max. Detrusordruck während Füllung: | 65 cm H₂O |

Hierzu kann auch auf Fig. 3B verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hypersensitiven, hypokapazitären, *low compliance* Harnblase vor.

Da die Detrusordrücke unverändert 40 cm H₂O überschreiten sowie unverändert eine Blase mit *low compliance* (Norm > 20 ml / cm H₂O) nachgewiesen werden kann und im klinischen Alltag als auch unter Messung unverändert Harninkontinenz auftritt, wird die Oxybutynindosis auf 4 x 10 mg gesteigert. Hierunter liegt nunmehr ein Sistieren der Dranginkontinenz tagsüber mit Anwendung einer Sicherheitsvorlage sowie nachts unverändert mit Anwendung einer Sicherheitswindel infolge seltener Inkontinenz in den frühen Morgenstunden vor.

Die Verlaufs-Videourodynamik nach drei Monaten 4x10 mg flüssigem Oxybutynin zeigt:

| | |
|---|---|
| erster Harndrang / korrespondierender Detrusordruck: | 50 ml / 5 cm H₂O |
| starker Harndrang / korrespondierender Detrusordruck: | - / - |
| maximales Füllvolumen / korrespondierender Detrusordruck: | 385 ml / 38 cm H₂O |
| Detrusorinstabilität ab: | Keine, *low compliance* von 10 ml / cm H₂O mit linearem Druckanstieg |
| max. Detrusordruck während Füllung: | 38 cm H₂O |

Es liegt zudem ein Reflux in distale Harnleiter bei 350 ml Füllung (= Reflux ersten Grades) vor.

Insgesamt kann hierzu kann auch auf Fig. 3C verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hypersensitiven, normo-hypokapazitären, *low compliance* Harnblase vor.

Der beidseitig auftretende Reflux ersten Grades ist dabei neuro-urologisch akzeptabel, und zwar auch deshalb, weil die Harnwegsinfekte auf ein Ereignis pro Jahr reduziert werden und diese nicht mehr fieberhaft verlaufen, sondern über Geruchsbildung und Trübung des Urins von der Patientin erkannt werden. Eine Kontroll-Szintigrafie der Nieren nach zwölf Monaten zeigt zudem einen prozentualen Anstieg der renalen Filtrationsleistung von 10 % als Ausdruck einer dauerhaften normwertigen intravesikalen Drucksituation. Die unveränderte *low compliance* der Blase ist daher klinisch akzeptabel, verlangt jedoch eine mindestens einmal pro Jahr vorzunehmende urodynamische Kontrolle und zudem eine alle anderthalb bis zwei Jahre durchzuführende Nierenszintigrafie-Kontrolle. Mit zunehmendem Alter können auch erneute Versuche einer zusätzlichen Einnahme von Anticholinergika erwogen werden.

### 4. Fallbericht IV:

### Patientin mit Multipler Sklerose als neurogene Grunderkrankung

Bei einer 62-jährigen Patientin mit Multipler Sklerose vom sekundär chronisch progredienten Verlauf besteht infolge einer beinbetonten Paraspastik eine Rollatorpflicht. Seit mindestens 2,5 Jahren liegen ein starker Harndrang mit Toilettengängen im 75-Minuten-Takt bis 90-Minuten-Takt tagsüber (bei Trinkmengenreduktion auf unter 1 Liter / d) sowie mindestens zweimal nachts und eine alltägliche Dranginkontinenz bei verkürzter Vorwarnzeit (= nOABwet) vor, wobei mindestens drei große Vorlagen tagsüber und mindestens eine Schutzhose in der Nacht als aufsaugende Schutzmittel eingesetzt werden. Der Urologe hat in der Vergangenheit 3 x 15 mg Trospiumchlorid / d zur Therapie verschrieben. Hierdurch mildgradige Symptomreduktion, jedoch traten nach bereits zwei bis drei Wochen stärkste Nebenwirkungen mit Schwindel, Sturzneigung und trockener Kehle auf. Ein gleiches Ergebnis resultierte nach alternativer Einnahme von retardiertem Propiverin (45 mg 1 x 1 Tablette / d). Seither wurde keine Theapie mehr der neurogenen Blasenfunktionsstörung durchgeführt. Es liegt eine deutliche eingeschränkte Lebensqualität mit drohender sozialer Isolation infolge der Pollakisurie und Dranginkontinenz vor.

Die initiale Urodynamik ohne jedwede Therapie der Blasenfunktionsstörung zeigt:

| | |
|---|---|
| erster Harndrang / korrespondierender Detrusordruck: | 50 ml / 35 cm H₂O |
| starker Harndrang / korrespondierender Detrusordruck: | 120 ml / 58 cm H₂O |
| maximales Füllvolumen / korrespondierender Detrusordruck: | 175 ml / 55 cm H₂O |
| Detrusorinstabilität ab: | 45 ml |
| max. Detrusordruck während Füllung: | 58 cm H₂O, Druckplateau um 55 cm H₂O undulierend |
| Compliance | Low-Compliance-Situation von 3,2 ml / cm H₂O |

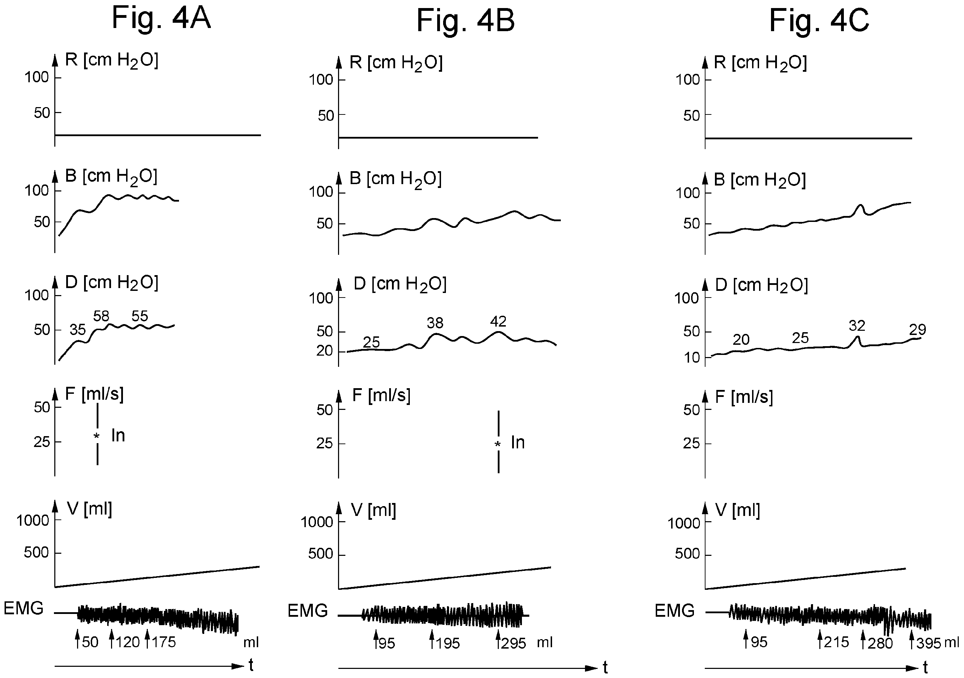

Hierzu kann auch auf Fig. 4A verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hypersensitiven, hypokapazitären, instabilen, hypertonen, *low-compliance* Harnblase vor.

Da die beinbetonte Paraspastik neurologischerseits gut unterdrückt bzw. eingestellt ist bei guter Hand- und Augenfunktion wird der intermittierende Selbstkatheterismus etabliert und hiernach eine Instillationstherapie mit 3 x 10 mg flüssigem Oxybutynin eingeleitet (entspricht 3 x 10 mg Oxybutynin / d). Hierunter liegen eine deutliche Symptomlinderung mit Reduktion der Miktionsfrequenz auf acht Toilettengänge tagsüber (bei normalisierter Flüssigkeitsaufnahme) und einmal nachts und eine Reduktion der Dranginkontinenzepisoden / Ereignisse sowie des Vorlagenverbrauchs um 50 % vor. Das Therapieergebnis wird ohne die unter oraler Therapie beklagten anticholinergen Nebenwirkungen erzielt.

Die Kontroll-Urodynamik unter 3 x 10 mg flüssigem Oxybutynin zeigt:

| | |
|---|---|
| erster Harndrang / korrespondierender Detrusordruck: | 95 ml / 25 cm H₂O |
| starker Harndrang / korrespondierender Detrusordruck: | 195 ml / 38 cm H₂O |
| maximales Füllvolumen / korrespondierender Detrusordruck: | 295 ml / 38 cm H₂O |
| Detrusorinstabilität ab: | 155 ml, ab 180 ml um 40 cm H₂O undulierend |
| max. Detrusordruck während Füllung: | 42 cm H₂O |
| Compliance | Low-Compliance-Situation von 16,5 ml / cm H₂O |

Hierzu kann auch auf Fig. 4B verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hypersensitiven, hypokapazitären, instabilen, normo- bis hypertonen, *low compliance* Harnblase vor.

Die potentiell nierengefährdenden Detrusordrücke konnten noch nicht unter 40 cm H₂O gesenkt werden, es besteht weiterhin eine Low-Compliance-Situation, wenngleich die Instillationstherapie zu einer deutlich gesteigerten Detrusornachgiebigkeit (von 3,2 auf 16,5 ml / cm H2O) führt. Zur Therapieoptimierung wird die Oxybutynindosis auf 5 x 10 ml gesteigert. Die Patientin ist infolge des bereits erzielten Therapieerfolges hochmotiviert, hierfür die tägliche Katheterisierungsfrequenz zu steigern. Hierunter liegen eine weitere Zunahme der Symptomlinderung mit Reduktion der Miktionsfrequenz auf sechs Toilettengänge tagsüber (bei weiterhin normalisierter Flüssigkeitsaufnahme) und einmal nachts, eine Reduktion der Dranginkontinenzepisoden bzw. Ereignisse sowie des Vorlagenverbrauchs um weitere 30 % vor. Das Therapieergebnis wird unverändert ohne die unter oraler Therapie beklagten anticholinergen Nebenwirkungen erzielt; allenfalls liegt eine nuancielle Obstipationsneigung vor, die mit Macrogoleinnahme im 2-Tagesrhythmus bis 3-Tagesrhythmus vermieden werden kann.

Die Kontroll-Urodynamik unter 5 x 10 mg flüssigem Oxybutynin zeigt:

| | |
|---|---|
| erster Harndrang / korrespondierender Detrusordruck: | 95 ml / 20 cm H₂O |
| starker Harndrang / korrespondierender Detrusordruck: | 215 ml / 25 cm H₂O |
| maximales Füllvolumen / korrespondierender Detrusordruck: | 395 ml / 29 cm H₂O |
| Detrusorinstabilität ab: | 280 ml |
| max. Detrusordruck während Füllung: | 32 cm H₂O |
| Compliance | normwertige Compliance-Situation von 20,3 ml / cm H₂O |

Hierzu kann auch auf Fig. 4C verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hyper- bis normsensitiven, hypokapazitären, stabilen, normotonen Harnblase vor.

### 5. Fallbericht V:

### Patient mit senso-motorisch inkompletter Querschnittlähmung unterhalb des siebten Halswirbelkörpers

Bei einem 44-jährigen Patienten mit senso-motorisch inkompletter Querschnittlähmung unterhalb des siebten Halswirbelkörpers infolge eines Arbeitsunfalles vor 8 Monaten (Montagearbeiter, Gerüststurz aus ca. 4,5 Meter Höhe) resultiert eine Tetraplegie ohne Beatmungspflichtigkeit mit kombinierter neurogener Blasen-, Mastdarm- und Sexualfunktionsstörung. Der intermittierende Selbstkatheterismus wird seit zwei Monaten beherrscht. Die ersten zwei Urodynamiken im Querschnittzentrum der versorgenden Unfallklinik hatten die Entwicklung einer überaktiven, *low compliance* Harnblase offenbart, welche mit Auftitration eines oralen Anticholinergikums behandelt wurde. Zuletzt benötigte der Patient 3 x 30 mg Spasmex®, um eine urodynamisch akzeptable Detrusordämpfung sowie Harnkontinenz im Alltag zu erzielen. Hierunter kam es zu einer nicht beherrschbaren Obstipation, welche auch nicht durch Irrigationen (Darmspülungen) im 2-Tagesrhythmus in Kombination mit Laxantien (oralen Abführmittel und Abführzäpfchen) und Laxativen (Stuhlweichmacher) zu beherrschen war. Das gleiche ungünstige Nutzen/Nebenwirkungs-Profil trat unter alternativen oralen Anticholinergika auf (4 x 5mg Oxybutynin oral). Seit einer Woche daher keine Blasentherapie mehr, d.h. im Sinne eines Auslassversuches zur Statuserhebung vor Einleitung alternativer Therapieoptionen. Hierunter ist der Patient alltäglich inkontinent trotz Steigerung der Katheterisierungsfrequenz auf bis zu 12 x / d.

Die Verlaufs-Urodynamik acht Monate nach Unfalltrauma unter Therapiekarenz hinsichtlich der Blasenfunktionsstörung seit einer Woche zeigt:

| | |
|---|---|
| erster Harndrang / korrespondierender Detrusordruck: | 50 ml / 40 cm H₂O |
| starker Harndrang / korrespondierender Detrusordruck: | 95 ml / 75 cm H₂O |
| maximales Füllvolumen / korrespondierender Detrusordruck: | 100 ml / 73 cm H₂O |
| Detrusorinstabilität ab: | mit Füllbeginn, etwa alle 95 ml bis 100 ml Reflexinkontinenz (komplette Inkontinenz / Blasenentleerung) |
| max. Detrusordruck während Füllung: | 80 cm H₂O |
| Compliance | Low-Compliance-Situation von unter 2 ml / cm H₂O |

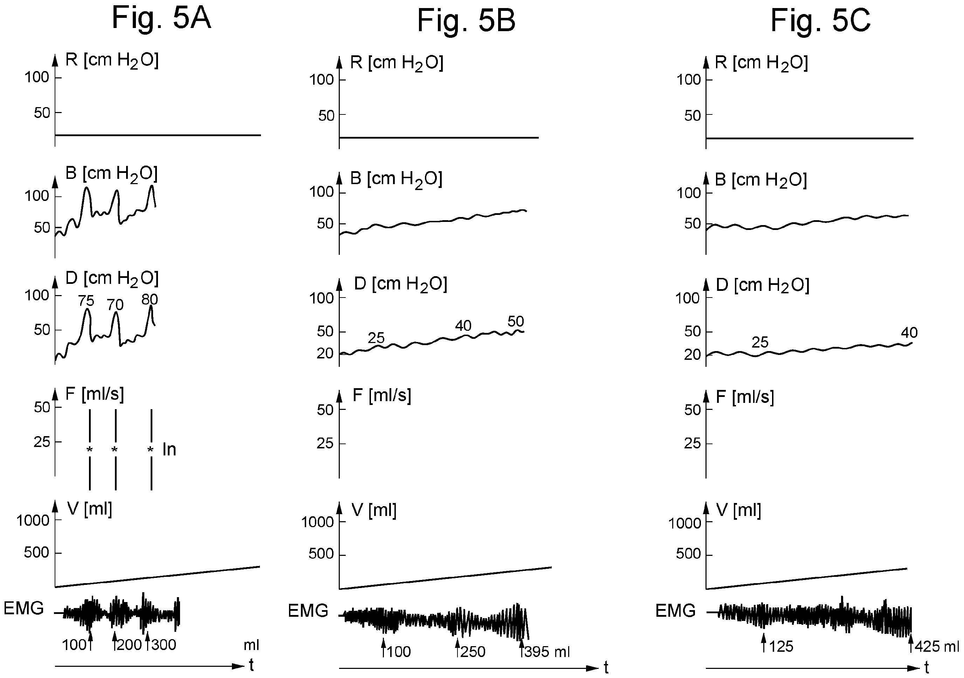

Hierzu kann auch auf Fig. 5A verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hypersensitiven, hypokapazitären, instabilen, hypertonen, *low compliance* Harnblase vor.

Als Therapieeskalation werden dem Patienten die Injektion von Botulinumtoxin-A in die Blasenwand oder alternativ die alltägliche Instillation von initial 4 x 10ml (entspricht 4 x 10mg) flüssigem Oxybutynin angeboten. Da die Botulinumtoxin-Injektion in Abhängigkeit der Dosis statistisch im 6-Monatsintervall bis 9-Monatsintervall wiederholt werden muss und der Patient sich bereits katheterisieren kann, entscheidet er sich zunächst für die Instillationstherapie. Hierunter sistiert die Inkontinenz vollständig, die Kathetersierungsfrequenz kann auf 6 x / d normalisiert werden, und die Darmfunktion ist unter Einnahme von Movicol® und Anwendung von Abführzäpfchen regelmäßig und ohne klinisch relevante Obstipation.

Die Verlaufs-Urodynamik einen Monat nach Beginn der Instillationstherapie mit 4 x 10 ml flüssigem Oxybutynin zeigt:

| | |
|---|---|
| erster Harndrang / korrespondierender Detrusordruck: | 100 ml / 25 cm H₂O |
| starker Harndrang / korrespondierender Detrusordruck: | 250 ml / 40 cm H₂O |
| maximales Füllvolumen / korrespondierender Detrusordruck: | 395 ml / 50 cm H₂O |
| Detrusorinstabilität ab: | ab Füllbeginn |
| max. Detrusordruck während Füllung: | 50 cm H₂O |
| Compliance | Low-Compliance-Situation von 13,2 ml / cm H₂O |

Hierzu kann auch auf Fig. 5B verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hypersensitiven, hypokapazitären, instabilen, hypertonen, *low compliance* Harnblase vor.

Die klinisch optimierte Situation spiegelt sich in einer deutlichen Reduktion des maximalen Detrusordruckes und in einem Anstieg der Detrusorcompliance wider. Beide Parameter sind jedoch noch optimierungswürdig, um den oberen Harnstrakt dauerhaft vor Folgeschäden zu schützen. Es erfolgt daher eine Dosissteigerung auf 6 x 10 ml / d flüssiges Oxybutynin.

Die Verlaufs-Urodynamik einen Monat nach Dosissteigerung der Instillationstherapie auf 6 x 10 ml flüssiges Oxybutynin zeigt:

| | |
|---|---|
| erster Harndrang / korrespondierender Detrusordruck: | 125 ml / 25 cm H₂O |
| starker Harndrang / korrespondierender Detrusordruck: | 280 ml / 30 cm H₂O |
| maximales Füllvolumen / korrespondierender Detrusordruck: | 425 ml / 40 cm H₂O |
| Detrusorinstabilität ab: | ab 50 ml |
| max. Detrusordruck während Füllung: | 40 cm H₂O |
| Compliance | normwertige Compliance-Situation von 21,25 ml / cm H₂O |

Hierzu kann auch auf Fig. 5C verwiesen werden (wobei zu den diesbezüglichen Abkürzungen auf die Ausführungen zu Fig. 1A bzw. Fig. 2A verwiesen werden kann).

Zusammenfassend liegt somit eine neurogene Blasenfunktionsstörung im Sinne einer hyper- bis normsensitiven, hypokapazitären, instabilen, normotonen Harnblase mit normwertiger Detrusorcompliance vor.

Im Ergebnis zeigen somit die zuvor angeführten Fallberichte, dass im Rahmen der vorliegenden Erfindung bei der Behandlung neurogener Blasenfunktionsstörungen insgesamt optimierte Behandlungsdosen aufgefunden und bestimmt werden können, und zwar auf Basis objektiver physiologisch-physikalischer Kenngrößen bzw. Messdaten bzw. -parameter und in weiterführender Abstimmung mit weiteren Kenngrößen, was erstmals eine optimierte und objektivierte Therapiefindung ermöglicht, einhergehend mit einem entsprechenden Therapieerfolg.

## Patentansprüche

1. Oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird,
wobei die Zusammensetzung das Oxybutynin bevorzugt in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutynin, bevorzugt Oxybutyninhydrochlorid (Oxybutynin-HCI), in einer Konzentration von (1 ± 0,5) mg/ml und
- mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,5) mg/ml
enthält,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient *(Compliance)* und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion.

2. Zusammensetzung zur Verwendung nach Anspruch 1,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck als Zielparameter unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder mit der Maßgabe und/oder Zielsetzung, dass der Detrusorkoeffizient *(Compliance)* als Zielparameter unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

3. Zusammensetzung zur Verwendung nach Anspruch 1 oder 2,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck als Zielparameter unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und mit der Maßgabe und/oder Zielsetzung, dass der Detrusorkoeffizient *(Compliance)* als Zielparameter unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

4. Zusammensetzung zur Verwendung nach einem der vorangehenden Ansprüche,
wobei der Detrusordruck bestimmt und/oder berechnet wird als Differenz aus intravesikalem Druck (Harnblasendruck) einerseits und Rektaldruck (Abdominaldruck) andererseits, insbesondere wobei der intravesikale Druck (Harnblasendruck) mittels urodynamischer Messung bestimmt wird, vorzugsweise während der insbesondere kontinuierlichen Harnblasenfüllung (Füllphase) der urodynamischen Messung, und/oder insbesondere wobei der Rektaldruck (Abdominaldruck) mittels (Druck-)Messung bestimmt wird, vorzugsweise während der insbesondere kontinuierlichen Harnblasenfüllung (Füllphase) der urodynamischen Messung; und/oder
wobei der Detrusorkoeffizient *(Compliance)* bestimmt und/oder berechnet wird als Quotient aus der Füllungsvolumenzunahme, insbesondere während der insbesondere kontinuierlichen Harnblasenfüllung (Füllphase), einerseits und dem korrelierenden intravesikalen Druckanstieg (Anstieg des Harnblasendrucks) anderseits, insbesondere wobei der intravesikale Druck (Harnblasendruck) mittels urodynamischer Messung bestimmt wird, vorzugsweise während der insbesondere kontinuierlichen Harnblasenfüllung (Füllphase) der urodynamischen Messung; und/oder
wobei der Detrusordruck als maximaler Detrusordruck, insbesondere maximaler Detrusordruck p_{Detrusor, max,} bestimmt wird, insbesondere bestimmt mittels urodynamischer Messung, vorzugsweise während der insbesondere kontinuierlichen Harnblasenfüllung (Füllphase) der urodynamischen Messung.

5. Zusammensetzung zur Verwendung nach einem der vorangehenden Ansprüche,
wobei die Behandlungsdosis, insbesondere die tagesbezogene Therapiedosis, bezogen auf das Oxybutynin, bevorzugt Oxybutyninhydrochlorid, für den Fall, dass der Detrusordruck unter Therapie mit der oxybutyninhaltigen Zusammensetzung mehr als 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder für den Fall, dass der Detrusorkoeffizient (*Compliance*) unter Therapie mit der oxybutyninhaltigen Zusammensetzung weniger als 25 ml / cm H₂O (25 Milliliter pro Zentimeter Wassersäule), insbesondere weniger als 20 ml / cm H₂O (20 Milliliter pro Zentimeter Wassersäule), beträgt, die Behandlungsdosis, insbesondere die tagesbezogene Therapiedosis, bezogen auf das Oxybutynin, bevorzugt Oxybutyninhydrochlorid, angepasst, insbesondere erhöht wird, vorzugsweise mit der/den zuvor in Anspruch 2 oder 3 definierten Maßgabe(n) und/oder Zielsetzung(en); und/oder
wobei die Behandlungsdosis, insbesondere die tagesbezogene Therapiedosis, bezogen auf das Oxybutynin, bevorzugt Oxybutyninhydrochlorid, für den Fall, dass der Detrusordruck unter Therapie mit der oxybutyninhaltigen Zusammensetzung mehr als 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und für den Fall, dass der Detrusorkoeffizient (*Compliance*) unter Therapie mit der oxybutyninhaltigen Zusammensetzung weniger als 25 ml / cm H₂O (25 Milliliter pro Zentimeter Wassersäule), insbesondere weniger als 20 ml / cm H₂O (20 Milliliter pro Zentimeter Wassersäule), beträgt, die Behandlungsdosis, insbesondere die tagesbezogene Therapiedosis, bezogen auf das Oxybutynin, bevorzugt Oxybutyninhydrochlorid, angepasst, insbesondere erhöht wird, vorzugsweise mit den zuvor in Anspruch 2 oder 3 definierten Maßgaben und/oder Zielsetzungen.

6. Zusammensetzung zur Verwendung nach einem der vorangehenden Ansprüche,
wobei (i) die urodynamische Kenngröße(n) (Messgröße(n)) ausgewählt ist bzw. sind aus der Gruppe von Detrusordruck und Detrusorkoeffizient (*Compliance*) sowie deren Kombination; und/oder
wobei (i) die urodynamische Kenngröße(n) (Messgröße(n)) mindestens eine weitere urodynamische Kenngröße (Messgröße) (i) umfasst bzw. umfassen, wobei die weitere urodynamische Kenngröße ausgewählt ist aus der Gruppe von maximaler Harnblasenkapazität, insbesondere zystometrischer maximaler Harnblasenkapazität, Harnblasenfüllungssensitivität und Gefühl des ersten Harndrangs sowie deren Kombinationen; und/oder
die urodynamische Kenngrößen (Messgrößen) ausgewählt sind aus der Gruppe von Detrusordruck und Detrusorkoeffizient (*Compliance*) sowie deren Kombination und zudem mindestens eine weitere urodynamische Kenngröße (Messgröße) (i) umfasst bzw. umfassen, wobei die weitere urodynamische Kenngröße ausgewählt ist aus der Gruppe von maximaler Harnblasenkapazität, insbesondere zystometrischer maximaler Harnblasenkapazität, Harnblasenfüllungssensitivität und Gefühl des ersten Harndrangs sowie deren Kombinationen.

7. Zusammensetzung zur Verwendung nach einem der vorangehenden Ansprüche,
wobei die Behandlungsdosis, insbesondere die tagesbezogene Therapiedosis, bezogen auf das Oxybutynin, bevorzugt Oxybutyninhydrochlorid, in Abhängigkeit von einer Kombination mindestens der nachfolgend angeführten klinischen, medizinischen und/oder anamnestischen patientenindividuellen Kenngrößen (i) bis (iii) und ggf. (iv) und (v) des zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen; insbesondere ausgewählt aus der Gruppe von Detrusordruck und Detrusorkoeffizient (*Compliance*) sowie deren Kombination;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) ggf. Art und/oder Schwere der pathologischen Blasenanatomie;
(v) ggf. Status der Nierenfunktion.

8. Zusammensetzung zur Verwendung nach einem der vorangehenden Ansprüche,
wobei die neurogenen Blasenfunktionsstörungen mit einem erhöhten Detrusordruck einhergehen und/oder hierdurch gekennzeichnet sind,
insbesondere wobei der Detrusordruck im unbehandelten Zustand und/oder ohne Therapie mit der oxybutyninhaltigen Zusammensetzung mehr als 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung.

9. Zusammensetzung zur Verwendung nach einem der vorangehenden Ansprüche,
zur topischen Applikation, insbesondere Instillation, in die Harnblase; und/oder
wobei die Zusammensetzung zur topischen Applikation, insbesondere Instillation, in die Harnblase, hergerichtet ist bzw. wobei die Zusammensetzung mittels topischer Applikation, insbesondere Instillation, in die Harnblase verabreicht wird; und/oder
wobei die Zusammensetzung sterilisiert, insbesondere dampfsterilisiert, bevorzugt wasserdampfsterilisiert, ist.

10. Zusammensetzung zur Verwendung nach einem der vorangehenden Ansprüche,
wobei die Zusammensetzung, insbesondere anwendungs-, dosier- und/oder applikationsfertig, in einer Aufbewahrungs- und/oder Applikationsvorrichtung, vorzugsweise Kolbenspritze, bevorzugt Einweg-Kolbenspritze, insbesondere zur topischen Applikation, insbesondere Instillation, in die Harnblase, vorliegt und/oder eingebracht ist;
insbesondere wobei die Aufbewahrungs- und/oder Applikationsvorrichtung, vorzugsweise die Kolbenspritze, bevorzugt die Einweg-Kolbenspritze, ein Volumen, insbesondere ein Aufnahmevolumen für die Zusammensetzung, im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, aufweist; und/oder
insbesondere wobei die Aufbewahrungs- und/oder Applikationsvorrichtung, vorzugsweise die Kolbenspritze, bevorzugt die Einweg-Kolbenspritze, die Zusammensetzung in einer Menge im Bereich von 1 ml bis 50 ml, insbesondere im Bereich von 2 ml bis 25 ml, vorzugsweise im Bereich von 3 ml bis 20 ml, bevorzugt im Bereich von 3,5 ml bis 15 ml, besonders bevorzugt im Bereich von 4 ml bis 12 ml, ganz besonders bevorzugt von etwa 5 ml oder ganz besonders bevorzugt von etwa 10 ml, aufweist; und/oder
insbesondere wobei die Aufbewahrungs- und/oder Applikationsvorrichtung, vorzugsweise die Kolbenspritze, bevorzugt die Einweg-Kolbenspritze, sterilisiert, insbesondere dampfsterilisiert, bevorzugt wasserdampfsterilisiert, ist, bevorzugt mitsamt der in der Aufbewahrungs- und/oder Applikationsvorrichtung vorliegenden und/oder eingebrachten Zusammensetzung; und/oder
insbesondere wobei die Aufbewahrungs- und/oder Applikationsvorrichtung aus einem Cycloolefin-(Co-)Polymer (COP bzw. COC) gebildet ist; und/oder insbesondere wobei die Aufbewahrungs- und/oder Applikationsvorrichtung als Kolbenspritze, bevorzugt Einweg-Kolbenspritze, ausgebildet ist und/oder in Form einer Kolbenspritze, bevorzugt Einweg-Kolbenspritze, vorliegt, insbesondere wobei die Kolbenspritze einen Spritzenkörper, insbesondere Spritzenzylinder, einerseits und einen Spritzenkolben mit einem Kolbenstopfen andererseits aufweist, wobei der Spritzenkörper aus einem Cycloolefin-(Co-)Polymer (COP bzw. COC) gebildet ist und wobei der Kolbenstopfen aus einem Halogenbutylkautschuk, insbesondere aus einem Chlorbutylkautschuk oder aus einem Brombutylkautschuk, vorzugsweise aus einem Brombutylkautschuk, gebildet ist.

11. Oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird, insbesondere Zusammensetzung nach einem der vorangehenden Ansprüche,
wobei die Zusammensetzung das Oxybutynin bevorzugt in Form des Hydrochlorids enthält, die Zusammensetzung als wässrige Zusammensetzung ausgebildet ist und die Zusammensetzung in Kombination und jeweils in wirksamen, insbesondere pharmazeutisch wirksamen Mengen,
- Oxybutynin, bevorzugt Oxybutyninhydrochlorid (Oxybutynin-HCI), in einer Konzentration von (1 ± 0,5) mg/ml und
- mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,5) mg/ml
enthält,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder mit der Maßgabe und/oder Zielsetzung, dass der Detrusorkoeffizient (*Compliance*) unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

12. Oxybutyninhaltige Zusammensetzung zur Verwendung bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, wobei die Zusammensetzung mittels topischer Verabreichung, insbesondere mittels Instillation, in die Harnblase appliziert wird, insbesondere Zusammensetzung nach einem der vorangehenden Ansprüche,
wobei die Zusammensetzung das Oxybutynin bevorzugt in Form des Hydrochlorids enthält, die Zusammensetzung als wässrige Zusammensetzung ausgebildet ist und die Zusammensetzung in Kombination und jeweils in wirksamen, insbesondere pharmazeutisch wirksamen Mengen,
- Oxybutynin, bevorzugt Oxybutyninhydrochlorid (Oxybutynin-HCI), in einer Konzentration von (1 ± 0,4) mg/ml, insbesondere in einer Konzentration von (1 ± 0,3) mg/ml, vorzugsweise in einer Konzentration von (1 ± 0,2) mg/ml, bevorzugt in einer Konzentration von (1 ± 0,1) mg/ml, besonders bevorzugt in einer Konzentration von etwa 1 mg/ml, und
- mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,4) mg/ml, insbesondere in einer Konzentration von (3,5 ± 0,3) mg/ml, vorzugsweise in einer Konzentration von (3,5 ± 0,2) mg/ml, bevorzugt in einer Konzentration von (3,5 ± 0,1) mg/ml, besonders bevorzugt in einer Konzentration von etwa 3,5 mg/ml,
enthält,
wobei die Zusammensetzung einen pH-Wert im Bereich von 3 bis 5, insbesondere im Bereich von 3,8 bis 4,5, aufweist, insbesondere wobei der pH-Wert der Zusammensetzung unter Verwendung von und/oder mittels Salzsäure eingestellt ist,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion,
wobei die Behandlungsdosis, insbesondere die tagesbezogene Therapiedosis, bezogen auf das Oxybutynin, bevorzugt Oxybutyninhydrochlorid, festgelegt und/oder bestimmt wird mit der Maßgabe und/oder Zielsetzung, dass der Detrusordruck unter Therapie mit der oxybutyninhaltigen Zusammensetzung höchstens 40 cm H₂O (40 Zentimeter Wassersäule) beträgt, insbesondere bestimmt mittels urodynamischer Messung, vorzugsweise während der insbesondere kontinuierlichen Harnblasenfüllung (Füllphase) der urodynamischen Messung, und/oder mit der Maßgabe und/oder Zielsetzung, dass der Detrusorkoeffizient (*Compliance*) unter Therapie mit der oxybutyninhaltigen Zusammensetzung mindestens 20 ml / cm H₂O (20 Milliliter pro Zentimeter Wassersäule), insbesondere mindestens 25 ml / cm H₂O (25 Milliliter pro Zentimeter Wassersäule), beträgt, insbesondere bestimmt mittels urodynamischer Messung, vorzugsweise während der insbesondere kontinuierlichen Harnblasenfüllung (Füllphase) der urodynamischen Messung.

13. Aufbewahrungs- und/oder Applikationsvorrichtung, vorzugsweise Kolbenspritze, bevorzugt Einweg-Kolbenspritze, vorzugsweise zur topischen Applikation, insbesondere Instillation, in die Harnblase, enthaltend eine oxybutyninhaltige Zusammensetzung nach einem der vorangehenden Ansprüche.

14. Verpackungseinheit, enthaltend mindestens eine Verpackung und mindestens eine Aufbewahrungs- und/oder Applikationsvorrichtung, vorzugsweise Kolbenspritze, bevorzugt Einweg-Kolbenspritze, wie in Anspruch 13 definiert, wobei die Aufbewahrungs- und/oder Applikationsvorrichtung, vorzugsweise die Kolbenspritze, bevorzugt die Einweg-Kolbenspritze, in die Verpackung eingebracht ist und/oder in der Verpackung vorliegt.

15. Kit, insbesondere Applikations- und/oder Instillationssystem, umfassend:
(I) mindestens eine in einer Aufbewahrungs- und/oder Applikationsvorrichtung, vorzugsweise Kolbenspritze, bevorzugt Einweg-Kolbenspritze, insbesondere wie in Anspruch 13 definiert, vorliegende oxybutyninhaltige Zusammensetzung, wie in einem der Ansprüche 1 bis 12 definiert;
(II) (a) mindestens eine an die Aufbewahrungs- und/oder Applikationsvorrichtung, vorzugsweise Kolbenspritze, bevorzugt Einweg-Kolbenspritze, anschließbare Applikations- und/oder Instillationsvorrichtung, insbesondere in Form eines Instillationsschlauches oder dergleichen, vorzugsweise in Form eines (Harnblasen-)Katheters, und/oder (b) mindestens eine an die Aufbewahrungs- und/oder Applikationsvorrichtung, vorzugsweise Kolbenspritze, bevorzugt Einweg-Kolbenspritze, anschließbare Anschlussvorrichtung, insbesondere Adapter, vorzugsweise zum Anschließen an die Applikations- und/oder Instillationsvorrichtung, insbesondere in Form eines Instillationsschlauches oder dergleichen, vorzugsweise in Form eines (Harnblasen-)Katheters;
und gegebenenfalls (III) mindestens eine Applikations- und/oder Instillationsanleitung.

16. Verfahren zur Bestimmung und/oder Festlegung einer Behandlungsdosis, insbesondere einer tagesbezogenen Therapiedosis, von Oxybutynin, insbesondere Oxybutyninhydrochlorid, als Wirkstoff bei der prophylaktischen und/oder therapeutischen Behandlung von insbesondere neurogenen Blasenfunktionsstörungen, insbesondere von neurogenen Blasenentleerungsstörungen, vorzugsweise von mit einer Detrusorhyperaktivität und/oder einer Detrusor-Sphinkter-Dyssynergie einhergehenden neurogenen Blasenfunktionsstörungen, bevorzugt des Syndroms der insbesondere neurogen bedingten überaktiven Harnblase, unter Verwendung einer oxybutyninhaltigen Zusammensetzung, insbesondere wie in einem der Ansprüche 1 bis 12 definiert, wobei die Zusammensetzung mittels topischer Verabreichung, insbesondere mittels Instillation, in die Harnblase appliziert wird,
wobei die Zusammensetzung das Oxybutynin bevorzugt in Form des Hydrochlorids enthält, die oxybutyninhaltige Zusammensetzung als wässrige Zusammensetzung ausgebildet ist und die oxybutyninhaltige Zusammensetzung in Kombination und jeweils in wirksamen, insbesondere pharmazeutisch wirksamen Mengen,
- Oxybutynin, bevorzugt Oxybutyninhydrochlorid (Oxybutynin-HCI), in einer Konzentration von (1 ± 0,5) mg/ml und
- mindestens ein Elektrolytsalz, bevorzugt Natriumchlorid, berechnet als Elektrolytkation, bevorzugt berechnet als Natrium im Fall von Natriumchlorid, in einer Konzentration von (3,5 ± 0,5) mg/ml
enthält,
wobei die Behandlungsdosis, insbesondere die tagesbezogene Therapiedosis, bezogen auf das Oxybutynin, bevorzugt Oxybutyninhydrochlorid, in Abhängigkeit von mindestens einem, insbesondere von einer Kombination von mindestens zwei, der nachfolgend angeführten klinischen, medizinischen und/oder anamnestischen patientenindividuellen Kenngrößen (i) bis (v) eines zu behandelnden Patienten bestimmt und/oder festgelegt wird:
(i) urodynamische Kenngröße(n) (Messgröße(n)) des zu behandelnden Patienten, insbesondere ausgewählt aus der Gruppe von Detrusordruck, Detrusorkoeffizient (*Compliance*) und intravesikalem Druck (Harnblasendruck) sowie deren Kombinationen;
(ii) Art und/oder Schwere der der neurogenen Blasenfunktionsstörung zugrundeliegenden und/oder der die neurogene Blasenfunktionsstörung hervorrufenden bzw. verursachenden Grunderkrankung des zu behandelnden Patienten;
(iii) Alter des zu behandelnden Patienten;
(iv) Art und/oder Schwere der pathologischen Blasenanatomie; und/oder
(v) Status der Nierenfunktion.
